# EUROPEAN PATENT APPLICATION

(11) **EP 4 796 120 A1**
(43) Date of publication of application: **26.08.2026**
(21) Application number: 24879745.8
(22) Date of filing: 16.10.2024
(51) Int. Cl.: A61K 38/17, A61K 38/18, A61P 9/00, A61P 9/04, A61P 9/10, A61P 43/00, C07K 14/47, C12N 15/12, C12N 15/85

(54) **METHOD FOR INDUCING MYOCARDIAL REGENERATION**

(30) Priority: 17.10.2023 JP 2023179125
(71) Applicant: National Cerebral and Cardiovascular Center, Suita-shi, Osaka 564-8565 (JP)
(72) Inventor: KIKUCHI, Kazu, Suita-shi, Osaka 564-8565 (JP); OGAWA, Masahito, Suita-shi, Osaka 564-8565 (JP)
(74) Representative: Weickmann & Weickmann PartmbB
(86) International application number: PCT/JP2024/036852
(87) International publication number: WO 2025/084323

(57) **Abstract**

The object of the present invention is to provide a novel method for inducing myocardial regeneration. The above-mentioned problem is solved by providing a method for inducing myocardial regeneration, which includes administering a therapeutically effective amount of FOXO6 to cardiomyocytes or inducing the expression of FOXO6 in cardiomyocytes.

## Description

### [Technical Field]

The present invention relates to a method for inducing myocardial regeneration, a method for treating heart disease, a composition for inducing myocardial regeneration, and the like, using FOXO6.

### [Background Art]

In the heart of adult mammals, cardiomyocytes, which are essential for the pumping function of the heart, are terminally differentiated cells that have completed their cell cycle and have limited proliferative capacity. As a result, the death of cardiomyocytes directly leads to a decline in heart pumping function, and becomes a major cause of high mortality and poor prognosis in heart disease. For example, in myocardial infarction, cardiomyocyte occlusion causes necrosis of the cardiomyocyte tissue. However, in the human heart, the repair capacity through regeneration of cardiomyocytes (myocardial regeneration) is extremely limited. Therefore, necrotic cardiomyocytes cannot regenerate and are replaced by fibrous scar tissue. Scar tissue lacks contractile function and cannot restore normal electrical conduction with the remaining cardiomyocytes. This leads to a decline in heart pumping function and the development of arrhythmias, and ultimately causes a poor prognosis that results in heart failure and sudden death.

Incidentally, recent studies have reported that mammalian hearts, like amphibians and fish, regenerate cardiomyocytes during the first few days after birth, and it has become clear that this regeneration is due to the dedifferentiation and proliferation of cardiomyocytes. Although the regenerative mechanism inherent in mammalian hearts becomes weak with growth, it is suggested to be maintained at a low level throughout life also in human hearts, and the development of a new myocardial regeneration therapy that reactivates this regenerative mechanism is expected. For example, the present inventors previously reported that KLF (Kruppel-like transcription factor) (e.g., KLF1, KLF2b) is effective in myocardial regeneration (Patent Literature 1).

### [Citation List]

### [Patent Literature]

[Patent Literature 1]
WO 2021/016663

### [Summary of Invention]

### [Technical Problem]

Although the technology described in Patent Literature 1 is superior, a technology for myocardial regeneration therapy with higher treatment effects has been desired.

Therefore, the present invention aims to provide a novel method for inducing myocardial regeneration.

### [Solution to Problem]

The present inventors have conducted intensive studies in an attempt to solve the above-mentioned problems and found that, in a system using zebrafish, the foxo6b gene strongly induces dedifferentiation of cardiomyocytes and is effective in inducing myocardial regeneration. Furthermore, the present inventors have found that co-expression of the foxo6b gene with a cell cycle activator (e.g., the ccnd1 gene (cyclin D)) increases the proliferation of cardiomyocytes, and completed the present invention.

That is, one embodiment of the present invention relates to the following.
[1] A composition for inducing myocardial regeneration comprising FOXO6 as an active ingredient.
[2] The composition of [1], wherein the aforementioned FOXO6 is administered in a therapeutically effective amount to cardiomyocytes.
[3] The composition of [2], wherein the aforementioned cardiomyocytes are those of an infant, child, or adult.
[4] The composition of [2] or [3], wherein the aforementioned administration is performed in vitro.
[5] The composition of [2] or [3], wherein the aforementioned cardiomyocytes are present in a subject.
[6] The composition of [5], wherein the aforementioned FOXO6 is administered to the aforementioned subject.
[7] The composition of [5] or [6], wherein the aforementioned FOXO6 is administered to the heart of the subject.
[8] The composition of any of [5] to [7], wherein the aforementioned myocardial regeneration promotes cardiac regeneration in the aforementioned subject.
[9] The composition of [8], wherein the aforementioned heart regeneration is characterized by an increase in ejection fraction, fractional shortening, or both.
[10] The composition of [8] or [9], wherein the aforementioned heart regeneration is characterized by an increase in vascular endothelial cells, epicardial cells, or both.
[11] The composition of any of [2] to [10], wherein the aforementioned FOXO6 induces dedifferentiation of the aforementioned cardiomyocytes to produce proliferative cardiomyocytes.
[12] The composition of [11], further comprising differentiating a population of the aforementioned proliferative cardiomyocytes to produce a population of cardiomyocytes.
[13] The composition of [12], wherein the aforementioned differentiation occurs in the substantial absence of the aforementioned FOXO6 or after the induction of the aforementioned FOXO6 has ceased.
[14] The composition of any of [1] to [13], wherein the aforementioned FOXO6 is FOXO6 protein, FOXO6 nucleic acid, or a vector containing FOXO6 nucleic acid.
[15] The composition of [14], wherein the aforementioned FOXO6 protein is a protein comprising an amino acid sequence that is at least 80% identical to the amino acid sequence shown in SEQ ID NO: 1, SEQ ID NO: 3, SEQ ID NO: 5, or SEQ ID NO: 7.
[16] The composition of [14], wherein the aforementioned FOXO6 nucleic acid is a nucleic acid that comprises or consists of a base sequence that is at least 80% identical to the base sequence shown in SEQ ID NO: 2, SEQ ID NO: 4, SEQ ID NO: 6, or SEQ ID NO: 8.
[17] The composition of [14], wherein the aforementioned vector comprises FOXO6 nucleic acid operatively bound to a promoter.
[18] The composition of [17], wherein the aforementioned promoter is a cardiac-specific promoter.
[19] The composition of [18], wherein the aforementioned cardiac-specific promoter is selected from the group consisting of an α-myosin heavy chain (α-MHC) promoter, a myosin light chain 2 (MLC-2) promoter, a cardiac troponin C (cTnC) promoter, an NCX1 promoter, and a TNNT2 promoter.
[20] The composition of [17] or [18], wherein the aforementioned promoter is an inducible promoter.
[21] The composition of [20], wherein the aforementioned inducible promoter is selected from the group consisting of a tetracycline-inducible promoter, a steroid hormone-inducible promoter, a hypoxia-inducible promoter, a promoter specific to a region near a site of heart injury, or a promoter that responds to stress resulting from ischemia and reperfusion.
[22] The composition of any of [1] to [21], further comprising a cell cycle activator.
[23] The composition of any of [1] to [21], which is used in combination with a cell cycle activator.
[24] The composition of [22] or [23], wherein the aforementioned cell cycle activator is at least one selected from the group consisting of a growth factor, a growth factor receptor, an intracellular factor with a growth-promoting effect, a cell cycle factor, an anti-proliferation inhibitor, and a molecule that activates molecular mechanisms of these.
[25] The composition of [22] or [23], wherein the aforementioned cell cycle activator is at least one selected from the group consisting of NRG1, G-CSF, retinoic acid, TGF-β, IGF, PDGF, SHH, RLN3, FGF1, Periostin, Agrin, a GSK3-β inhibitor, and a Hippo pathway inhibitor.
[26] The composition of any of [11] to [25], wherein the aforementioned proliferative cardiomyocyte is a cardiomyocyte progenitor cell, an immature cardiomyocyte, or a cardiomyocyte having an embryonic phenotype.
[27] The composition of any of [1] to [26], wherein the aforementioned myocardial regeneration does not involve reprogramming of a cardiomyocyte cell lineage.
[28] The composition of any of [11] to [25], wherein the aforementioned proliferative cardiomyocyte re-enters the cell cycle.
[29] The composition of any of [5] to [28], wherein the aforementioned myocardial regeneration is characterized by an increase in the number of epicardial cells, vascular endothelial cells, or both.
[30] The composition of any of [5] to [29], wherein the aforementioned subject has heart disease associated with the loss of cardiomyocytes.
[31] The composition of [30], wherein the aforementioned heart disease is myocardial infarction, ischemic cardiomyopathy, dilated cardiomyopathy, or heart failure.
[32] A method for inducing myocardial regeneration, comprising administering a therapeutically effective amount of FOXO6 to cardiomyocytes or inducing the expression of FOXO6 in cardiomyocytes.
[33] The method of [32], further comprising administering a therapeutically effective amount of a cell cycle activator to cardiomyocytes.
[34] A population of cardiomyocytes or proliferative cardiomyocytes produced by the method of [32] or [33].
[35] A composition comprising cardiomyocytes, proliferative cardiomyocytes, or both produced by the method of [32] or [33].
[36] A method for treating heart disease, comprising administering a therapeutically effective amount of FOXO6 to a subject.
[37] Use of FOXO6 in the manufacture of a medicament for the treatment of heart disease.
[38] FOXO6 for use in the treatment of heart disease.
[39] A method for inducing dedifferentiation of cardiomyocytes, comprising administering FOXO6 to cardiomyocytes or inducing the expression of FOXO6 in cardiomyocytes.

### [Advantageous Effects of Invention]

According to the present invention, dedifferentiation of cardiomyocytes can be strongly induced, which enables efficient induction of myocardial regeneration. In addition, myocardial regeneration can be efficiently induced according to the present invention, which is useful in the treatment of heart disease.

### [Brief Description of Drawings]

[Fig. 1]

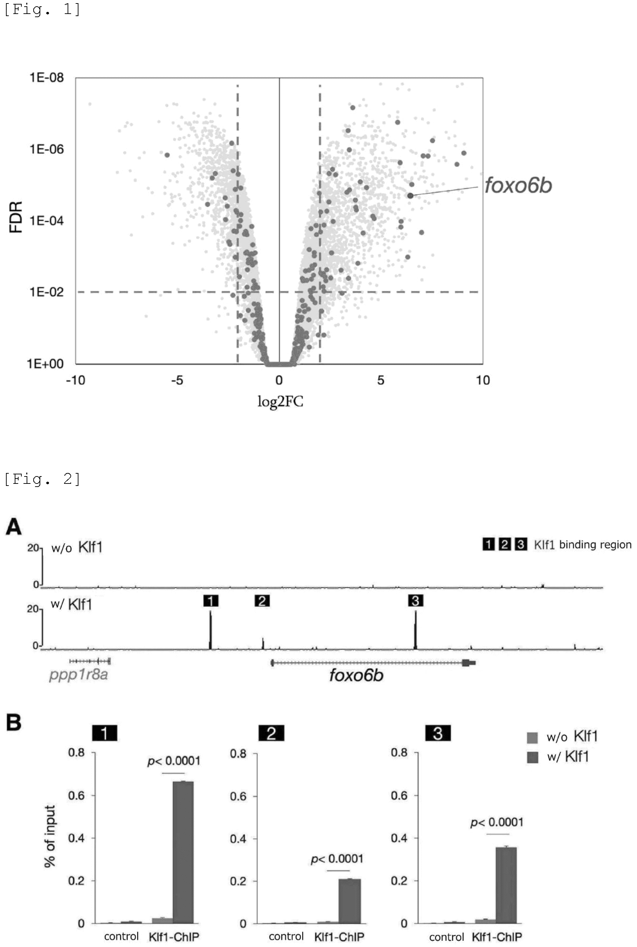

   Fig. 1 shows the results of a reanalysis of gene expression data from Klf1-overexpressing hearts (Ogawa et al. Science 2021). The vertical dotted line indicates log₂FC=2, and the horizontal dotted line indicates FDR=0.01.
[Fig. 2]
   Fig. 2 shows the results of ChIP-seq (Fig. 2A) and quantitative PCR analysis (Fig. 2B) regarding the regulation of foxo6b gene expression by Klf1.
[Fig. 3]

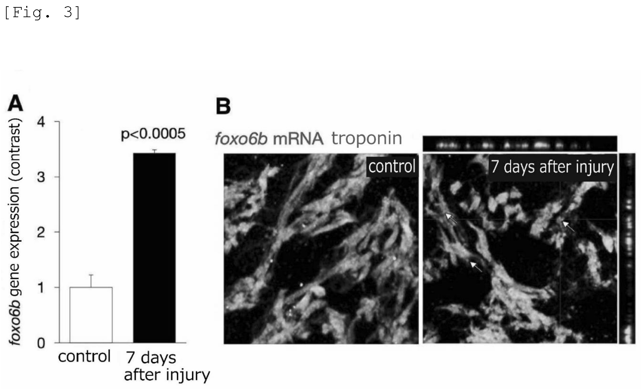

   Fig. 3 shows the results of quantitative PCR analysis (Fig. 3A) and in situ hybridization (Fig. 3B) regarding foxo6b gene expression in damaged zebrafish hearts.
[Fig. 4]

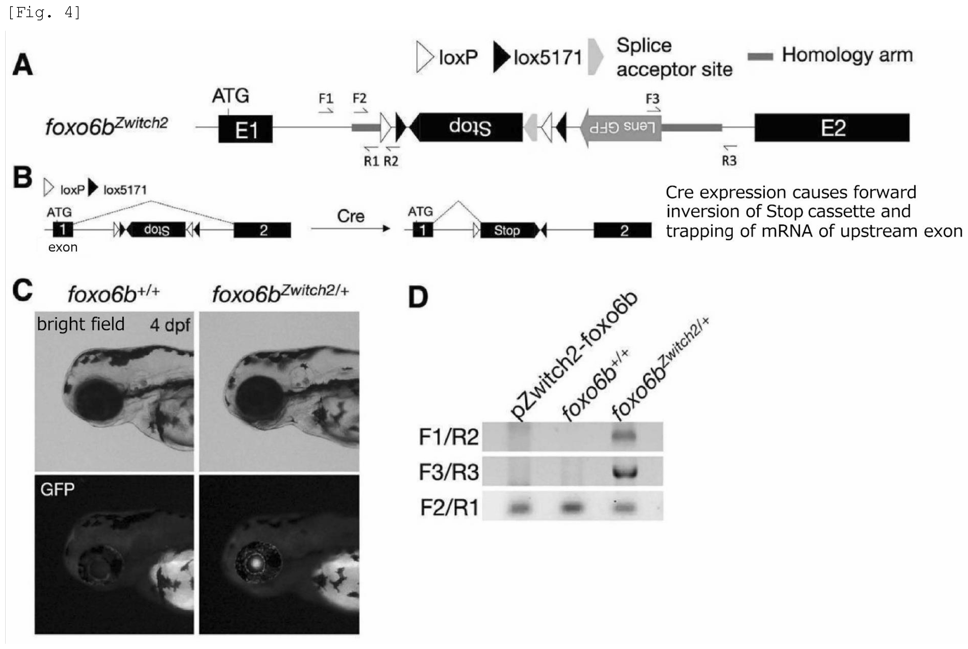

   Fig. 4A shows the expression repression cassette used in the production of a time- and tissue-specific foxo6b gene expression repression model. Fig. 4B is a schematic diagram showing the change in the splicing state of foxo6b mRNA due to Cre activity. Fig. 4C and Fig. 4D show the results of confirming the genomic insertion of the foxo6b gene expression repression cassette by GFP expression and genomic PCR.
[Fig. 5]

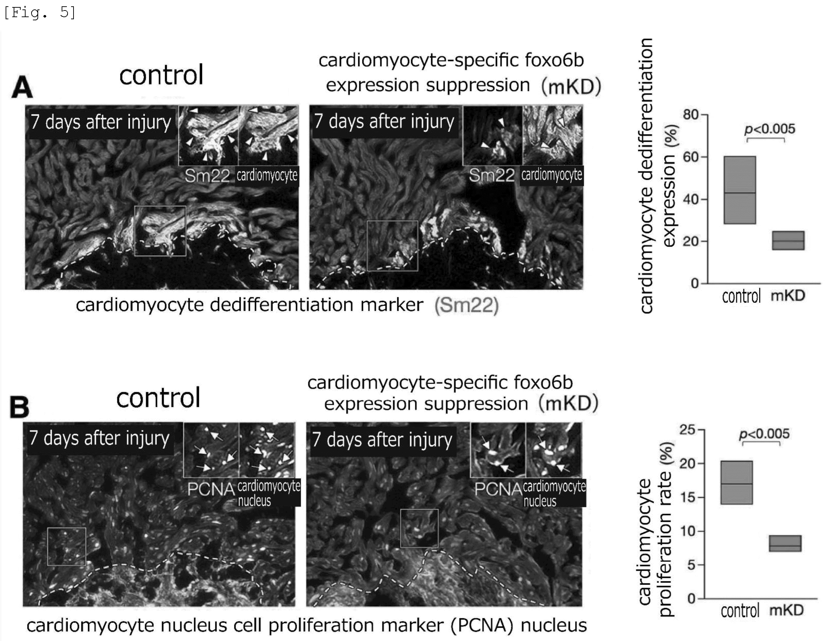

   Fig. 5 shows the results of immunofluorescence staining of dedifferentiation marker (Fig. 5A) and cell proliferation marker (Fig. 5B) in hearts in which foxo6b gene expression was specifically repressed in the cardiomyocyte.
[Fig. 6]

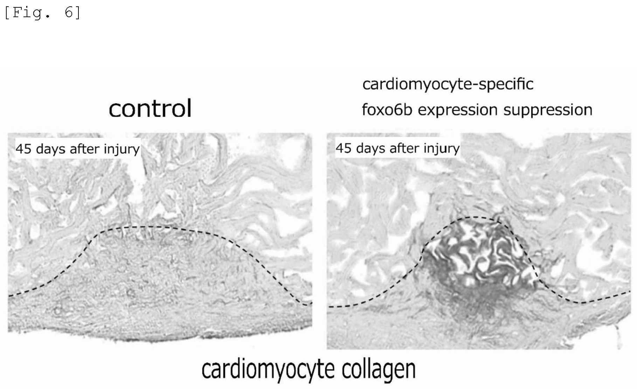

   Fig. 6 shows the results of Picro-Mallory staining in hearts in which foxo6b gene expression was specifically repressed in the cardiomyocyte.
[Fig. 7]

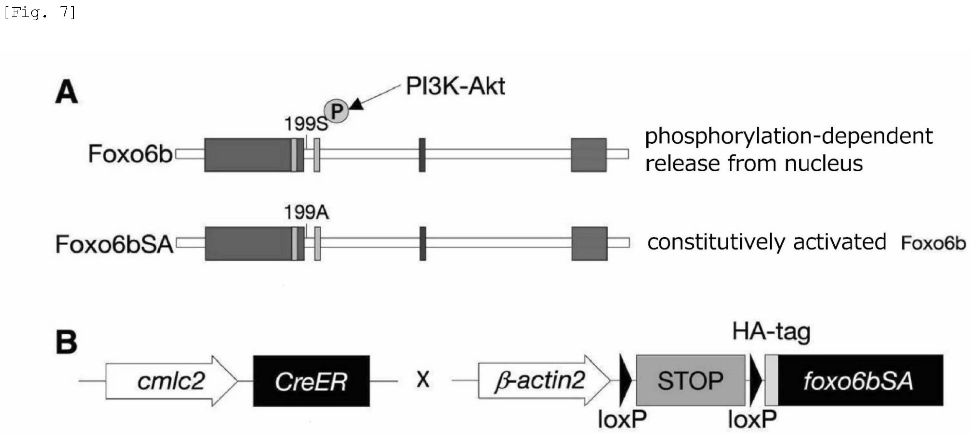

   Fig. 7A is a schematic diagram of foxo6bSA in the production of a zebrafish line expressing constitutively activated foxo6b. Fig. 7B is a schematic diagram of a zebrafish line crossed to express constitutively activated foxo6b specifically in the cardiomyocyte.
[Fig. 8]

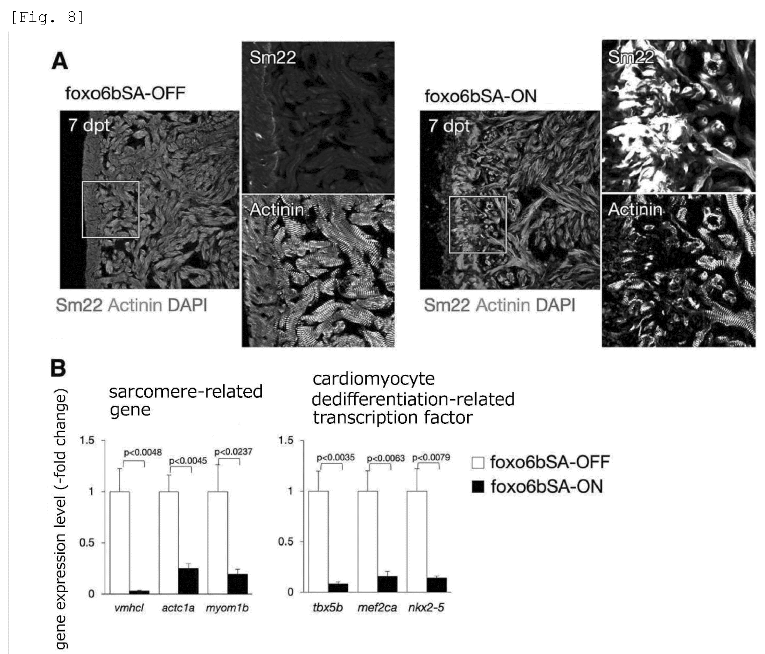

   Fig. 8 shows the results of immunofluorescence staining (Fig. 8A) and quantitative PCR analysis (Fig. 8B) performed on hearts expressing foxo6bSA myocardial-specifically.
[Fig. 9]

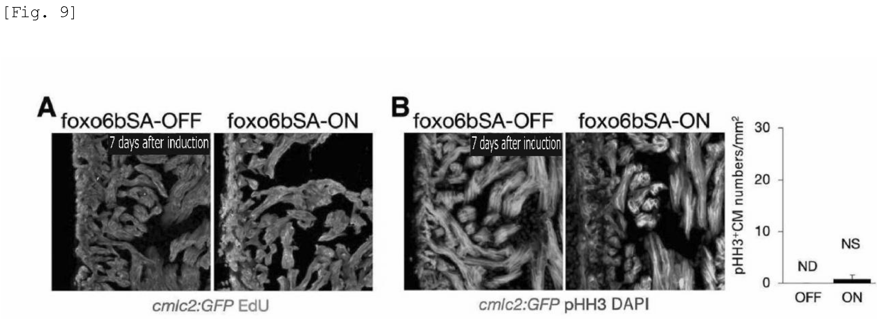

   Fig. 9 shows the results of immunofluorescence staining with EdU (Fig. 9A) and immunofluorescence staining with phosphorylated histone H3 (Fig. 9B) performed on hearts expressing foxo6bSA myocardial-specifically.
[Fig. 10]

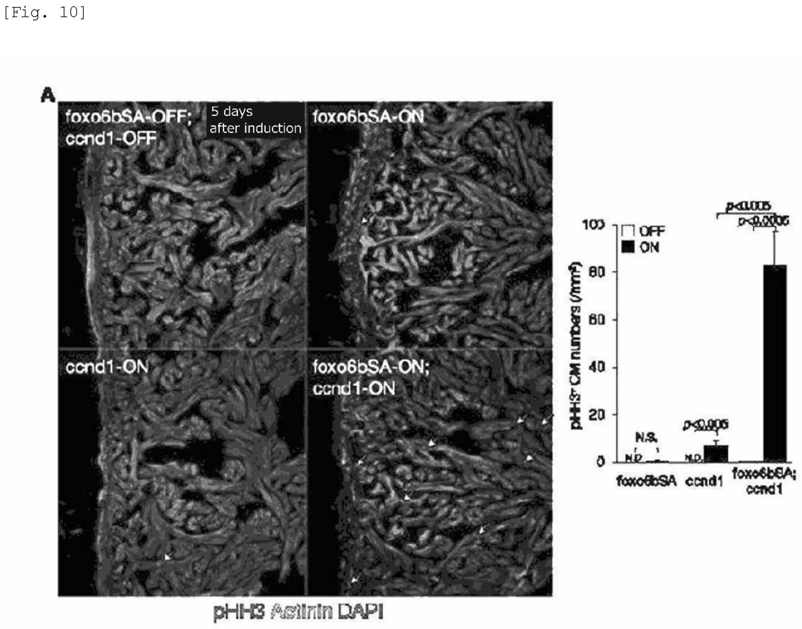

   Fig. 10 shows the results of immunofluorescence staining with phosphorylated histone H3 performed on hearts expressing foxo6bSA and ccnd1 myocardial-specifically.
[Fig. 11]

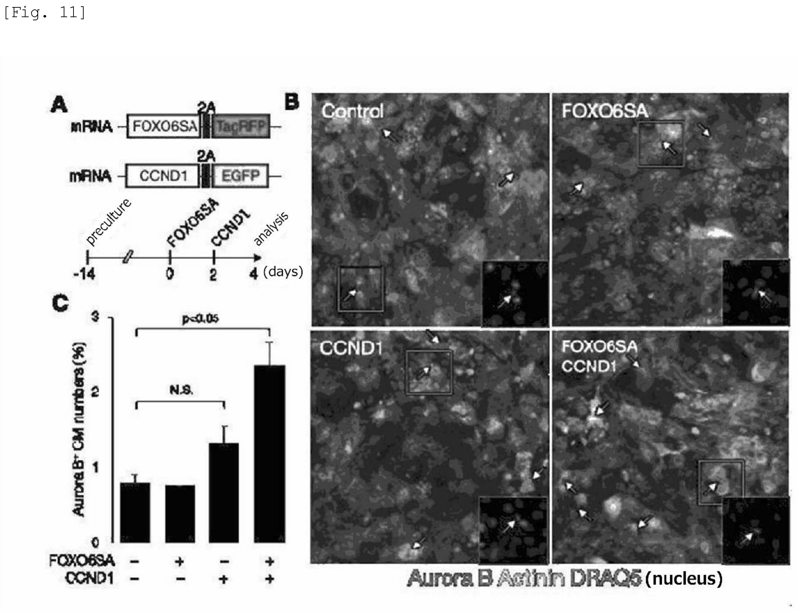

   Fig. 11 shows the results of immunofluorescence staining with Aurora kinase B in human iPS cell-derived cardiomyocytes expressing foxo6SA and ccnd1.

### [Description of Embodiments]

One example of the embodiments of the present invention is described in detail in the following, but the present invention is not limited thereto.

### (Summary of the present invention)

In one embodiment of the present invention, the following invention using FOXO6 is provided:
(1) A composition for inducing myocardial regeneration, comprising FOXO6 as an active ingredient.
(2) A method for inducing myocardial regeneration, comprising administering a therapeutically effective amount of FOXO6 to cardiomyocytes or inducing the expression of FOXO6 in cardiomyocytes.
(3) A method for treating heart disease comprising administering a therapeutically effective amount of FOXO6 to a subject.
(4) Use of FOXO6 in the manufacture of a medicament for the treatment of heart disease.
(5) FOXO6 for use in the treatment of heart disease.
(6) A method for inducing dedifferentiation of cardiomyocytes, comprising administering FOXO6 to cardiomyocytes or inducing the expression of FOXO6 in cardiomyocytes.

As described above, the development of a new myocardial regeneration induction technique with higher therapeutic effect that can be applied to myocardial regeneration therapy has been demanded. The present inventors have conducted intensive studies from the above-mentioned perspective and found for the first time that the foxo6b gene strongly induces dedifferentiation of cardiomyocytes in systems using zebrafish and is effective in inducing myocardial regeneration. The cardiomyocyte dedifferentiation ability of foxo6b is stronger than that of KLF (e.g., KLF1, KLF2b) previously found by the present inventors, and cannot be predicted at all from conventional findings.

The present invention is described in detail below, and any explanation given in any of the above-mentioned sections (1) to (6) is quoted as the explanation of other sections.

### (Definition)

When used in the present specification, unless otherwise specified, "FOXO6" is intended to include the FOXO6 protein and FOXO6 nucleic acid of any animal species. Therefore, when simply referred to as "FOXO6," it may include, for example, human-derived, mouse-derived, or zebrafish-derived FOXO6 protein, FOXO6 nucleic acid, and the like.

In the present specification, "treatment" means obtaining a desired pharmacological and/or physiological effect. The effect may be prophylactic from the viewpoint of completely or partially preventing the disease or symptoms thereof, or therapeutic from the viewpoint of partially or completely curing the disease and/or side effects caused by the disease. "Treatment" refers to any treatment of a disease in mammals, particularly humans, including, for example, the following: (a) preventing the onset of the disease in a subject who may be predisposed to the disease but has not yet been diagnosed with the disease; (b) suppressing the disease, i.e., preventing the onset thereof; and (c) alleviating the disease (i.e., causing regression of the disease).

In the present specification, "individual" and "subject" are used interchangeably and are not particularly limited. They refer to animals such as fish (e.g., zebrafish), rodents (e.g., rat, mouse), non-human primates, humans, dogs, cats, ungulates (e.g., horse, cow, sheep, pigs), birds (e.g., quail), and the like. In one embodiment of the present invention, "individual" and "subject" are preferably human.

In the present specification, "therapeutically effective amount" means an amount of active ingredient sufficient to effectively perform such treatment for a disease when administered to a mammal or other subject. "Therapeutically effective amount" varies depending on the active ingredient (compound, etc.) or cells, the disease and severity thereof, and the age, weight, etc., of the subject to be treated.

### (Method)

In one embodiment of the present invention, for example, increasing the level or activity of FOXO6 by administering at least one FOXO6 nucleic acid to cardiac tissue is useful for promoting or inducing myocardial regeneration in vitro or in vivo. Therefore, a method for promoting or inducing myocardial regeneration including administering at least one FOXO6 protein, or at least one FOXO6 nucleic acid, or at least one vector containing FOXO6 nucleic acid to a subject, and a composition therefor are provided. In one embodiment of the present invention, myocardial regeneration is associated with cardiomyocyte dedifferentiation, for example, dedifferentiation of adult cardiomyocytes.

Preferred subject includes individuals with heart disease (e.g., mammalian subjects such as humans; non-human primates; experimental non-human mammalian subjects such as mice and rats). Heart disease may result in ischemic cardiac tissue, for example, individuals with coronary artery disease; and the like. Suitable subjects include those with heart failure or degenerative heart diseases such as familial cardiomyopathy, dilated cardiomyopathy, hypertrophic cardiomyopathy, restrictive cardiomyopathy, or coronary artery disease resulting in ischemic cardiomyopathy.

The subjects may be infants, children, or adults.

In one embodiment of the present invention, the method includes administering FOXO6 protein, FOXO6 nucleic acid, or a vector containing FOXO6 nucleic acid to the target cardiomyocytes or cardiomyocyte tissue. Alternatively or additionally, the method may include inducing the expression of FOXO6 in the target cardiomyocytes. The addition or expression of active FOXO6 in cardiomyocytes dedifferentiates the cardiomyocytes. The obtained cells (proliferative cardiomyocytes) can then proliferate to replace at least some of the cardiomyocytes lost, for example, due to an ischemic event. After proliferation, the dedifferentiated cells redifferentiate into functional cardiomyocytes.

In one embodiment of the present invention, FOXO6 protein, FOXO6 nucleic acid, or a vector containing FOXO6 nucleic acid is administered to cardiomyocytes or cardiac tissue. Administration to cardiomyocytes and/or cardiac tissue may be performed in vitro or in vivo. The FOXO6 protein, FOXO6 nucleic acid, or a vector containing FOXO6 nucleic acid can be brought into direct contact with cells, that is, applied directly to cells, or, for example, the FOXO6 protein, FOXO6 nucleic acid, or a vector containing FOXO6 nucleic acid can be injected into the target bloodstream, thereby delivering the molecules to the cells and indirectly binding to the cells. Alternatively or additionally, the FOXO6 protein, FOXO6 nucleic acid, or a vector containing FOXO6 nucleic acid may be administered directly to the heart, for example, by injection into cardiomyocytes.

In these methods, a therapeutically effective amount of FOXO6 protein, FOXO6 nucleic acid, or a vector containing FOXO6 nucleic acid is administered to the subject. In one embodiment of the present invention, the administration includes direct delivery of FOXO6 protein, FOXO6 nucleic acid, or a vector containing FOXO6 nucleic acid to cardiac tissue or cardiomyocytes.

Administration of FOXO6 protein, FOXO6 nucleic acid, or a vector containing FOXO6 nucleic acid can be achieved by any method known in the art. In one embodiment of the present invention, contact between cells and FOXO6 or FOXO6 nucleic acid occurs in vitro or in vivo. The FOXO6 protein or FOXO6 nucleic acid may be brought into direct contact with cells, that is, directly applied to cardiomyocytes, or it may be indirectly bound to cells by, for example, injecting the FOXO6 protein or FOXO6 nucleic acid into the target cardiac tissue.

In one embodiment of the present invention, administration of FOXO6 protein, FOXO6 nucleic acid, or a vector containing FOXO6 nucleic acid increases the level of FOXO6 in cardiomyocytes or cardiac tissue compared to endogenous FOXO6 levels. In the present specification, "endogenous" used in this context means the "naturally occurring" level of FOXO6 expression and/or activity before administration of FOXO6 protein, FOXO6 nucleic acid, or a vector containing FOXO6 nucleic acid.

### (Myocardial regeneration)

Myocardial regeneration is a complex process in which cardiomyocytes (CMs), which form the muscle tissue (myocardium) of the heart, divide and create new cells. As disclosed in the present specification, FOXO6-induced myocardial regeneration is mediated by the reprogramming of CMs and expansion (proliferation) thereof, followed by redifferentiation into mature contractile CMs. In particular, FOXO6-induced myocardial regeneration is achieved by strongly inducing the dedifferentiation of cardiomyocytes, unlike KLF described in Patent Literature 1.

FOXO6 induces the dedifferentiation of cardiomyocytes and produces proliferative cardiomyocytes. That is, cardiomyocytes are terminally differentiated cells in normal adult individuals that are unable to differentiate to form or regenerate other cell types. Generally, it is recognized that, after birth, cardiomyocytes undergo terminal differentiation characterized by binucleation and centrosome degradation, and cardiac regeneration is not possible. However, as shown in the examples described later, administration of FOXO6 induces the dedifferentiation of CMs into proliferative CMs. In one embodiment of the present invention, the proliferative cardiomyocytes (CMs) are mitotic.

In one embodiment of the present invention, the present method includes proliferating proliferative cardiomyocytes in the presence of FOXO6. This may occur in vitro or in vivo under appropriate cell or tissue culture conditions. The result of the proliferation is that each CM produces a population of proliferative cardiomyocytes.

Once FOXO6 is removed, or metabolized or degraded, the population of proliferative cardiomyocytes spontaneously differentiates to produce a population of cardiomyocytes. In this way, FOXO6 can be used to increase the number of cardiomyocytes in vitro or in vivo.

In one embodiment of the present invention, CM expressing FOXO6 has a paracrine effect. For example, as illustrated in the present specification, FOXO6 expression can increase the number of epicardial cells and vascular endothelial cells.

In one embodiment of the present invention, under the control of the myosin heavy chain (MHC) promoter, the expression of FOXO6 in CM does not generally induce the expression of Raldh2 (retinal aldehyde dehydrogenase 2; also known as Aldhla2), which is a marker for epicardial and endothelial cells. Therefore, the cell lineage does not change even when FOXO6 is expressed in CM. Furthermore, the cell lineage does not change even after proliferation for 1, 2, 3, 4, 5, 6, 7, 8, 9, 10, 11, or 12 days. In one embodiment of the present invention, the expression of FOXO6 by the MHC promoter is at a lower level compared to normal CM. That is, FOXO6-induced myocardial regeneration in adult hearts does not involve reprogramming of the cell lineage. Rather, FOXO6-induced myocardial regeneration is characterized by reprogramming of the state of adult CM to a highly proliferative state, along with the ability to promote the growth of adjacent tissues.

### (FOXO6)

In the present specification, "FOXO6" means FOXO6 protein, FOXO6 nucleic acid, or variants or functional fragments thereof derived from any animal species (e.g., mammals such as mice, humans, and non-human primates, and non-mammals such as zebrafish, salamanders, and newts).

Examples of the FOXO6 protein include human-derived FOXO6 (SEQ ID NO: 1), mouse-derived Foxo6 (SEQ ID NO: 3), zebrafish-derived foxo6b (sometimes referred to as "Foxo6b") (SEQ ID NO: 5), zebrafish-derived foxo6a (sometimes referred to as "Foxo6a") (SEQ ID NO: 7), and the like.

Examples of the FOXO6 nucleic acid include human-derived FOXO6 (SEQ ID NO: 2), mouse-derived Foxo6 (SEQ ID NO: 4), zebrafish-derived Foxo6b (SEQ ID NO: 6), zebrafish-derived Foxo6a (SEQ ID NO: 8), and the like.

As described in the present specification, FOXO6 protein or nucleic acid encoding FOXO6 protein (FOXO6 nucleic acid) can be used to induce myocardial regeneration in vitro or in vivo. Therefore, FOXO6 protein and FOXO6 nucleic acid can be used to treat conditions in which dedifferentiation, proliferation, or both of cardiomyocytes are desirable, such as ischemic injury after myocardial infarction (MI); heart injury due to cardiotoxic agents (e.g., anthracycline antibiotics such as doxorubicin, cocaine, methamphetamine, cyclic antidepressants, calcium channel blockers, beta-blockers, and digoxin) or trauma (whether accidental or intentional as a result of surgery); heart failure; or age-related decline in cardiac function.

The methods disclosed in the present specification can be used for cardiac regeneration. In the present specification, "cardiac regeneration" means structural and/or functional regeneration or improvement of a damaged heart. For example, structural improvement may be an increase in the quantity or number of cardiomyocytes in the heart after administration of FOXO6 (including administration in combination with cell cycle activators). Examples of functional improvements include increased cardiac contractility or ejection fraction after FOXO6 administration (including administration in combination with cell cycle activators).

For example, ejection fraction, fractional shortening, or both can increase by 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, or more compared to the ejection fraction or fractional shortening before FOXO6 administration.

In one embodiment of the present invention, cardiac regeneration occurs within 2, 3, 4, 5, 6, 7, 8, 9, 10, 11, 12, 13, 14, 15, 16, 17, 18, 19, 20, 21, 22, 23, 24, 25, 26, 27, or 28 days after administration of FOXO6.

The method disclosed in the present specification can utilize FOXO6 variants or FOXO6 functional fragments. The variants or functional fragments can bind DNA with the same specificity as wild-type FOXO6 and retain at least one function of wild-type FOXO6. For example, in one embodiment of the present invention, the variant or functional fragment is any variant of FOXO6 that retains DNA-binding activity.

In one embodiment of the present invention, FOXO6 may be a conjugate or fusion protein. For example, it is a FOXO6 fusion protein. The fusion protein includes at least a portion of another protein, peptide, or polypeptide, for example, a nuclear localization sequence or an additional FOXO6 or domain thereof, for example, at least a portion of FOXO6 linked to a transactivation domain via a peptide bond. In one embodiment of the present invention, FOXO6 may be fused with a transactivation domain from another protein, for example, transactivation domain p53 or VP16. The fusion protein may also include a marker protein (e.g., fluorescent protein such as GFP) or a protein that assists isolation and/or purification (e.g., FLAG or His tag). The non-FOXO6 sequence may be the amino-terminus or carboxyl-terminus of the FOXO6 sequence.

In one embodiment of the present invention, FOXO6 is fused with one or more nuclear localization sequences.

In one embodiment of the present invention, the FOXO6 nucleic acid encodes a FOXO6 fusion protein.

In one embodiment of the present invention, the present method requires administration of a FOXO6 protein containing the amino acid sequence of a mature FOXO6 protein. Alternatively, the FOXO6 protein may be at least 80% identical to the amino acid sequence of a mature FOXO6 protein. Generally, FOXO6 useful for the method described in the present specification is at least 80% identical to the amino acid sequence of a wild-type FOXO6 protein, for example, 80, 81, 82, 83, 84, 85, 86, 87, 88, 89, 90, 91, 92, 93, 94, 95, 96, 97, 98, or 99% identical to the amino acid sequence of a wild-type FOXO6 protein. Useful FOXO6 proteins can be identified by conventional experiments. Generally, the FOXO6 protein functions as a wild-type FOXO6 protein.

This method involves administering a FOXO6 nucleic acid containing a mature FOXO6 protein coding sequence. Alternatively, the FOXO6 nucleic acid may be at least 80% identical to the nucleotide sequence encoding the mature FOXO6 protein. Generally, FOXO6 nucleic acids useful in the method described in the present specification are at least 80% identical to wild-type FOXO6 nucleic acid, for example, 80, 81, 82, 83, 84, 85, 86, 87, 88, 89, 90, 91, 92, 93, 94, 95, 96, 97, 98, or 99% identical to wild-type FOXO6 nucleic acid. Useful nucleic acids can be identified by routine experiments. Generally, it is desirable that the nucleic acid encodes a protein that functions as the wild-type FOXO6 protein.

In one embodiment of the present invention, the FOXO6 nucleic acid may be an ssRNA, dsRNA, dsDNA, or expression vector, for example, viral expression vector of a plasmid expression vector containing the FOXO6 nucleic acid. The FOXO6 nucleic acid may contain one or more modifications.

In one embodiment of the present invention, the FOXO6 nucleic acid contains at least one nucleotide modified at the 2' position of a sugar, for example, 2'-O-alkyl, 2'-O-alkyl-O-alkyl, or 2'-fluoro modified nucleotide. In other embodiments, RNA modification includes 2'-fluoro, 2'-amino, and 2'-O-methyl modifications on the ribose of pyrimidine, basic residue at the 3' end of RNA, or inverted base.

Many modifications of nucleotides and nucleosides make the nucleic acids into which they are incorporated more resistant to nuclease digestion. Specific examples of modified FOXO6 nucleic acids include those containing a modified skeleton, such as phosphorothioate, phosphotriester, methyl phosphonate, short-chain alkyl or cycloalkyl intersaccharide bond, or short-chain heteroatom or heterocyclic intersaccharide bond. In one embodiment of the present invention, modifications to the FOXO6 nucleic acid include a phosphorothioate skeleton or a heteroatom skeleton, particularly CH₂-NH-O-CH₂, CH, -N(CH₃)-O-CH₂ (known as the methylene (methylimino) or MMI skeleton), CH₂-O-N(CH₃)-CH₂, CH₂-N(CH₃)-N(CH₃)-CH₂ or O-N(CH₃)-CH₂-CH₂ skeleton (where the native phosphodiester skeleton is represented as O-P-O-CH); an amide skeleton; a morpholino skeleton structure; and a peptide nucleic acid (PNA) skeleton (where the phosphodiester skeleton of the oligonucleotide is substituted with a polyamide skeleton, and the nucleotide is directly or indirectly bonded to the aza nitrogen atom of the polyamide skeleton). Examples of phosphorus-containing bonds include, but are not limited to, phosphorothioates, chiral phosphorothioates, phosphorodithioates, phosphotriesters, aminoalkyl phosphotriesters, methyl and other alkyl phosphonates including 3'-alkylene phosphonates and chiral phosphonates, phosphinates, phosphoramides including 3'-aminophosphoramides and aminoalkyl phosphoramides, thionophosphoramides, thionoalkyl phosphonates, thionoalkyl phosphotriesters, and borane phosphates.

Modified nucleic acid skeletons that do not contain phosphorus atoms have a ck skeleton formed by a short-chain alkyl or cycloalkyl nucleoside bond, a mixed heteroatom and alkyl or cycloalkyl nucleoside bond, or one or more short-chain heteroatom or heterocyclic nucleoside bonds. These include morpholino bonds (partially formed from the sugar portion of nucleosides); siloxane skeletons; sulfide, sulfoxide, and sulfone skeletons; formacetyl and thioformacetyl skeletons; methyleneformacetyl and thioformacetyl skeletons; alkene-containing skeletons; sulfamate skeletons; methyleneimino and methylenehydrazino skeletons; sulfonate and sulfonamide skeletons; amide skeletons; and others having mixed N, O, S, and CH₂ components.

Furthermore, one or more substituted sugar moieties may include, for example, one of the following at the 2' position: OH, SH, SCH₃, F, OCN, OCH₃OCH₃, OCH₃O(CH₂)n CH₃, O(CH₂)n NH₂ or O(CH₂)n CH₃ (where n is 1 to about 10); C1-C10 lower alkyl, alkoxyalkoxy, substituted lower alkyl, alkalyl, or aralkyl; Cl; Br; CN; CF₃; OCF₃; O-, S-, or N-alkyl; O-, S-, or N-alkenyl; SOCH₃; SO₂; CH₃; ONO₂; NO₂; N₃; NH₂; heterocycloalkyl; heterocycloalkalyl; aminoalkylamino; polyalkylamino; substituted silyl; a group for improving the pharmacokinetic properties of nucleic acids; or a group for improving the pharmacodynamic properties of nucleic acids and other substituents having similar properties. As another example, nucleic acid sequences may include 2'-modified nucleotides such as 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAOE), or 2'-O-N-methylacetamide (2'-O-NMA).

As yet another example, the FOXO6 nucleic acid sequence may contain at least one 2'-O-methyl modified nucleotide, and in one embodiment of the present invention, all nucleotides contain 2'-O-methyl modification. In another embodiment, the modification is 2'-methoxyethoxy [2'-O-CH₂CH₂OCH₃, also known as 2'-O-(2-methoxyethyl)]. Other modifications include 2'-methoxy(2'-O-CH₃), 2'-propoxy(2'-OCH₂CH₃), 2'-fluoro(2'-F), and the like. Similar modifications can also be made at other positions on the nucleic acid, particularly at the 3' position of the sugar on the 3' terminal nucleotide and at the 5' position of the 5' terminal nucleotide. The nucleic acid may also have sugar mimetic groups such as cyclobutyl instead of the pentofuranosyl group.

The FOXO6 nucleic acid may also include additional or alternative base modification or substitution. As used herein, "unmodified" or "natural" nucleic acid bases include adenine (A), guanine (G), thymine (T), cytosine (C), and uracil (U). Modified nucleic acid bases include those that are rarely or transiently present in natural nucleic acids, such as hypoxanthine, 6-methyladenine, 5-Me pyrimidine, particularly 5-methylcytosine (also known as 5-methyl-2'deoxycytosine and often referred to in the art as 5-Me-C), 5-hydroxymethylcytosine (HMC), glycosyl HMC, and gentobiosyl HMC, as well as synthetic nucleic acid bases such as 2-aminoadenine, 2-(methylamino)adenine, 2-(imidazolylalkyl)adenine, 2-(aminoalkylamino)adenine, and other heterosubstituted alkyladenines, 2-thiouracil, 2-thiothymine, 5-bromouracil, 5-hydroxymethyluracil, 8-azaguanine, 7-deazaguanine, N6(6-aminohexyl)adenine, and 2,6-diaminopurine. Inosine may also be included. Other modifications include other synthetic and natural nucleic acid bases such as 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyluracil and 5-cytosine, 6-azouracil, 6-cytosine and 6-thymine, 5-uracil (pseudouracil), 4-thiouracil,, 8-halo, 8-amino, 8-thiol, 8-thioalkyl, 8-hydroxyl, or other 8-substituted adenines and 8-substituted guanines, 5-halo, particularly 5-bromo, 5-trifluoromethyl, or 5-substituted uracil and 5-substituted cytosine, 7-methylguanine and 7-methyladenine, 8-azaguanine and 8-azaadenine, 7-deazaguanine and 7-deazaadenine, 3-deazaguanine and 3-deazaadenine, and the like.

It is not necessary for all positions within a given nucleic acid to be uniformly modified. It is sufficient for more than one of the aforementioned modifications to be incorporated within a single oligonucleotide, or even within a single nucleoside within an oligonucleotide.

In one embodiment of the present invention, a nucleic acid is chemically linked to one or more sites or conjugates that enhance the activity, cell distribution, or cell uptake of an oligonucleotide. Such portions include, but are not limited to, cholesterol moiety, thioethers such as hexyl-S-tritylthiol, aliphatic chains such as dodecanediol or undecyl residue, phospholipids such as di-hexadecyl-rac-glycerol or triethylammonium 1,2-di-O-hexadecyl-rac-glycero-3-H-phosphonate, polyamine or polyethylene glycol chain, adamantane acetate, palmityl moiety, and octadecylamine or hexylaminocarbonyl-t-oxycholesterol moiety.

In one embodiment of the present invention, the FOXO6 nucleic acid is operatively bound to a promoter sequence. The promoter sequence may be constitutively active or compositionally active. In one embodiment of the present invention, the promoter sequence is cardiac-specific. For example, the cardiac-specific promoter sequence may be an alpha-myosin heavy chain (α-MHC) promoter, a myosin light chain 2 (MLC-2) promoter, a cardiac troponin C (cTnC) promoter, an NCX1 promoter, or a TNNT2 promoter.

In one embodiment of the present invention, the FOXO6 nucleic acid is operatively bound to an inducible promoter, such as tetracycline-inducible promoter, steroid hormone (e.g., progesterone or ecdysone)-inducible promoter, or hypoxia-responsive promoter.

In another embodiment, the FOXO6 nucleic acid is operatively bound to a promoter that is active or selectively active in a cardiac region adjacent to the injury site. Examples of such promoters include GATA-4 promoter.

In other embodiments, the FOXO6 nucleic acid is operatively bound to the promoter of a gene that is overexpressed in cardiac tissue in response to stress resulting from ischemia and reperfusion. These genes include aminoadipic acid semialdehyde synthase, apolipoprotein E, flavin-containing monooxygenase 2, NADPH oxidase 4, prostaglandin endoperoxide synthase 2, recombinant activating gene 2, stearoylcoenzyme A desaturase 1, solute carrier family 38 (member 1), and the like.

### (Administration of FOXO6)

In the methods described in the present specification, the FOXO6 protein or FOXO6 nucleic acid is administered to a subject. In one embodiment of the present invention, the FOXO6 protein or FOXO6 nucleic acid is administered to a target cell, tissue, or organ, or an expression vector encoding the FOXO6 nucleic acid is administered to a target cell, tissue, or organ in which the FOXO6 nucleic acid is expressed. In one embodiment of the present invention, the administration is systemic, and the expression vector is taken up by target cells, tissues, or organs. In one embodiment of the present invention, the expression vector may be taken up by non-target cells, tissues, or organs, but preferably, it does not have a serious adverse effect on such cells or tissues or the whole subject.

Methods for administering or delivering nucleic acids and expression constructs to target cells are known in the art, and include, for example, the methods described below. Target cells may be, for example, cardiomyocytes. In one embodiment of the present invention, FOXO6 protein, FOXO6 nucleic acid, or a vector containing FOXO6 nucleic acid is delivered in vivo to target cells, tissues, or organs. In one embodiment of the present invention, FOXO6 protein, FOXO6 nucleic acid, or a vector containing FOXO6 nucleic acid is administered to target cells ex vivo. In one embodiment of the present invention, FOXO6 protein, FOXO6 nucleic acid, or a vector containing FOXO6 nucleic acid is delivered to target cells in vitro.

In one embodiment of the present invention, the target cells are cardiomyocytes. The cardiomyocytes may be present within the target or in a culture outside the target. In one embodiment of the present invention, FOXO6 protein, FOXO6 nucleic acid, or a vector containing FOXO6 nucleic acid is administered to the heart or cardiac tissue.

In other embodiments, the target cells are proliferative cardiomyocytes. That is, FOXO6 protein, FOXO6 nucleic acid, or a vector containing FOXO6 nucleic acid can be administered to cardiomyocytes that have already undergone FOXO6-induced dedifferentiation.

In one embodiment of the present invention, a vector containing FOXO6 nucleic acid or FOXO6 nucleic acid can be transfected or transduced into target cells ex vivo. The transfected or transduced cells, capable of expressing FOXO6, can then be amplified and administered to a subject. In one embodiment of the present invention, the cells are of autologous origin to the subject. Suitable cells include those isolated from blood or bone marrow, such as adult hematopoietic stem/progenitor cells.

In one embodiment of the present invention, FOXO6 protein, FOXO6 nucleic acid, or a vector containing FOXO6 nucleic acid is delivered systemically, such as by intravenous injection. Additional administration route may include, for example, oral, topical, intracardiac, and intramuscular routes. In one embodiment of the present invention, FOXO6 protein, FOXO6 nucleic acid, or a vector containing FOXO6 nucleic acid can be delivered ex vivo to cells taken from a subject, and then cells containing the FOXO6 protein, FOXO6 nucleic acid, or vector containing FOXO6 nucleic acid can be reintroduced into the subject.

In one embodiment of the present invention, FOXO6 protein, FOXO6 nucleic acid, or a vector containing FOXO6 nucleic acid is administered via cardiac catheterization.

Numerous methods for the delivery of nucleic acids (FOXO6 nucleic acid, etc.) are known in the art. These methods include adeno-associated virus (AAV) or lentiviral-mediated delivery, nanoparticle-mediated delivery, gel-form-mediated intrapericardial delivery, and direct intramuscular administration of nucleic acid to the heart.

A preferred method for administering FOXO6 nucleic acid is by the use of adeno-associated virus (AAV). In one embodiment of the present invention, FOXO6 nucleic acid can be expressed continuously or conditionally. Furthermore, the use of cardiac-acting AAV serotypes or variants improves tissue specificity. Therefore, for example, one such method is the delivery of FOXO6 nucleic acid by cardiac-acting AAV. Suitable AAVs include cardiac-specific AAVs such as those described in Pacak and Byrne, Mol Ther, 2011, 19(9), pp1582-1590.

Other viruses, such as retrovirus, lentivirus, HSV, or adenovirus, can also be used.

By using a cardiac tissue-specific promoter (e.g., NCX1, TNNT2) for expression, further specificity can be obtained in addition to AAV serotype.

In one embodiment of the present invention, FOXO6 protein, FOXO6 nucleic acid, or a vector containing FOXO6 nucleic acid is administered by transfection using a transfection agent or a delivery vehicle. In the present specification, "delivery vehicle" means a compound that enhances the entry of FOXO6 nucleic acid into cells. Examples of delivery vehicles include protein-polymer complexes (polyplexes), polymer-lipid combinations (lipopolyplexes), multilayer and recharge particles, lipids and liposomes (lipoplexes, e.g., cationic liposomes and lipids), polyamines, calcium phosphate precipitates, polycations, histone proteins, polyethyleneimines, polylysine, and polyampholite complexes. In one embodiment of the present invention, the delivery vehicle includes a transfection agent. The transfection agent may be used to condense the nucleic acid. Transfection agents may also be used to associate functional groups with polynucleotides. Non-limiting examples of functional groups include cellular target sites, cell receptor ligands, nuclear localization signals, compounds that enhance the release of contents from endosomes or other intracellular vesicles (membrane-active compound, etc.), and other compounds that alter the behavior or interaction of the compound or complex to which they are bound (interaction modifiers). For in vivo delivery, complexes made with a sub-neutral amount of cationic transfection agent can be used.

In one embodiment of the present invention, FOXO6 nucleic acid or a vector containing FOXO6 nucleic acid can be delivered using exosomes or exosome-like vesicles. For example, FOXO6 nucleic acid may be introduced into exosome-producing cells, and exosomes containing FOXO6 nucleic acid may be isolated from those cells. Alternatively, exosomes may be isolated or prepared according to any method known in the art, and FOXO6 nucleic acid may be introduced into the exosomes.

In one embodiment of the present invention, FOXO6 nucleic acid or a vector containing FOXO6 nucleic acid can be delivered using lipopolymer, liposome, gelatin complex, poloxamine nanosphere, or lipoprotein.

An alternative technique for achieving cardiac-specific delivery of FOXO6 nucleic acid or a vector containing FOXO6 nucleic acid is ultrasound-targeted microbubble disruption (UTMD). This technique is based on the physical properties of ultrasound contrast agents, which are gas-filled microbubbles that vibrate to disruption when converted into sound waves by ultrasound. Transfection to the heart can be performed by loading FOXO6 nucleic acid or an expression vector into microbubbles, injecting them intravenously, and disrupting them in the heart by ultrasound.

In one embodiment of the present invention, FOXO6 nucleic acid or a vector containing FOXO6 nucleic acid can be delivered systemically. In one embodiment of the present invention, FOXO6 nucleic acid or a vector containing FOXO6 nucleic acid can be delivered in combination with one or more pharmaceutically acceptable carriers. Polymer reagents for delivering FOXO6 nucleic acid or a vector containing FOXO6 nucleic acid may incorporate a compound that enhances the usefulness thereof. These groups can be incorporated into the monomer before polymer formation or attached to the polymer after formation thereof. Vector transfer-enhancing moiety is a molecule that can modify the nucleic acid complex and guide it to any cellular location (tissue cell, etc.) or intracellular location (nucleus, etc.) in culture or in the whole organism. By altering the cellular or tissue location of the complex, the desired localization and activity of FOXO6 nucleic acid or a vector containing FOXO6 nucleic acid can be enhanced. Transfer-enhancing moieties can be, for example, protein, peptide, lipid, steroid, sugar, carbohydrate, nucleic acid, cell receptor ligand, or synthetic compound. In one embodiment of the present invention, a transfer-enhancing moiety can enhance cell binding to receptors, cytoplasmic transport to the nucleus, and nuclear entry or release from endosomes or other intracellular vesicles.

Nuclear localization signal (NLS) can also be used to enhance the targeting of the vector to the vicinity of the nucleus and/or entry into the nucleus. Such nuclear transport signal can be a protein or peptide, such as SV40 large-tagged NLS or nucleoplasmin NLS. These nuclear localization signals interact with various nuclear transport factors, such as NLS receptor (carioferrin alpha), and then with carioferrin beta. Nuclear transport proteins themselves can also function as NLS, since they are targeted to nuclear pores and the nucleus in one embodiment of the present invention.

Those skilled in the art will be able to select and use an appropriate system for delivering FOXO6 nucleic acid or a vector containing FOXO6 nucleic acid to the heart or cardiac tissue, or to cardiomyocytes or other target cells in vitro, ex vivo, or in vivo, without excessive experimentation.

In one embodiment of the present invention, local delivery of FOXO6 nucleic acid or a vector containing FOXO6 nucleic acid is desirable. In particular, delivery of FOXO6 nucleic acid or a vector containing FOXO6 nucleic acid to the heart is desirable.

There are numerous strategies to enable local delivery of FOXO6 protein, FOXO6 nucleic acid, or a vector containing FOXO6 nucleic acid to the heart. For example, osmotic minipumps, such as Alzet^{®} osmotic pump, can be used for effective local delivery of FOXO6 protein, FOXO6 nucleic acid, or a vector containing FOXO6 nucleic acid at sustainable therapeutic concentrations. Pumps with reservoirs are generally implanted in subcutaneous tissues and deliver FOXO6 protein, FOXO6 nucleic acid, or a vector containing FOXO6 nucleic acid to the target tissue via a silicone tube or cannula. Osmotic minipumps rely on osmosis to achieve steady-state drug delivery and are already in clinical use.

Local delivery of FOXO6 protein, FOXO6 nucleic acid, or a vector containing FOXO6 nucleic acid may also be achieved by cell transplantation. In addition to cell replacement therapy, cells containing FOXO6 protein, FOXO6 nucleic acid, or a vector containing FOXO6 nucleic acid may be transplanted into cardiomyocytes.

### (Dosage of FOXO6)

The effective dose level of the FOXO6 protein, FOXO6 nucleic acid, or vector containing FOXO6 nucleic acid to be administered may be appropriately determined depending on various factors such as the type and stage of the condition to be treated; the activity and properties of the FOXO6 protein, FOXO6 nucleic acid, or vector containing FOXO6 nucleic acid to be employed; the composition to be employed; the age, weight, general health, sex, and diet of the subject; the time of administration; the route of administration; the duration of treatment; drugs used in combination with or concurrently with the treatment; and other relevant factors well known in medicine.

Those skilled in the art can determine the effective and non-toxic dose required to treat applicable conditions, through routine experimentation.

Generally, the effective dose is expected to be in the range of about 0.0001 mg to about 1000 mg per kg body weight per 24 hr; typically, about 0.001 mg to about 750 mg per kg body weight per 24 hr; about 0.01 mg to about 500 mg per kg body weight per 24 hr; about 0.1 mg to about 500 mg per kg body weight per 24 hr; about 0.1 mg to about 250 mg per kg body weight per 24 hr; or about 1.0 mg to about 250 mg per kg body weight per 24 hr. More typically, the effective dose range is assumed to be in the range of about 10 mg to about 200 mg per kg body weight per 24 hr.

Alternatively, the effective dose may be up to about 5000 mg/m². Generally, the effective dose is assumed to be in the range of about 10 mg/m² to about 5000 mg/m², typically about 10 mg/m² to about 2500 mg/m², about 25 mg/m² to about 2000 mg/m², about 50 mg/m² to about 1500 mg/m², about 50 mg/m² to about 1000 mg/m², or about 75 mg/m² to about 600 mg/m².

Furthermore, it is apparent to those skilled in the art that the optimal amount and interval of individual doses are determined by the nature and degree of the condition to be treated, the form, route and site of administration, and the characteristics of the particular individual to be treated. Such optimal conditions can also be determined by conventional techniques.

Furthermore, it is apparent to those skilled in the art that the optimal treatment course, for example, the number of doses of the composition to be administered per day for a defined number of days, can be confirmed using a conventional course of treatment determination test.

Furthermore, the effectiveness of the treatment may be evaluated by determining the expression level of FOXO6 in samples from the subject treated with FOXO6 protein, FOXO6 nucleic acid, or a vector containing FOXO6 nucleic acid. After a certain period, the expression level of FOXO6 nucleic acid in further samples from the subject may be determined, and the change in the expression level of FOXO6 nucleic acid may indicate the effectiveness of the treatment plan. The samples may include plasma or serum.

Alternatively or additionally, the effectiveness of the treatment can be evaluated, for example, by determining the level of proliferative cardiomyocytes in samples from the subject treated with FOXO6 protein, FOXO6 nucleic acid, or a vector containing FOXO6 nucleic acid.

### (Composition)

In one embodiment of the present invention, a composition for inducing myocardial regeneration is provided.

The composition for inducing myocardial regeneration may contain FOXO6 as an active ingredient, as well as any other substance generally used in the pertinent technique field. Such substances are not particularly limited, and include pharmaceutically acceptable carriers, excipients, diluents, and the like.

### (Population of cardiomyocytes or proliferative cardiomyocytes)

Administration of FOXO6 protein, FOXO6 nucleic acid, or a vector containing FOXO6 nucleic acid generates a population of proliferative cardiomyocytes. The proliferative cardiomyocytes may be a population of immature cardiomyocytes, a population of cardiomyocytes or cardiomyocyte progenitor cells with an embryonic phenotype, or any combination of those. This population of proliferative cardiomyocytes can be incorporated into a pharmaceutical composition for administration to a subject. In one embodiment of the present invention, the population of proliferative cardiomyocytes can be differentiated into cardiomyocytes before being incorporated into the pharmaceutical composition.

The FOXO6 protein, FOXO6 nucleic acid, or expression vector can be administered to adult cardiomyocytes harvested from a subject (or other site) to induce myocardial regeneration. Whether the obtained cells are a population of cardiomyocytes or proliferative cardiomyocytes, these cells can be prepared as a pharmaceutical composition, for example, a sterile aqueous or non-aqueous solution, suspension, or emulsion, further containing a physiologically acceptable carrier (i.e., non-toxic substance that does not interfere with the activity of cardiomyocytes). Any suitable carrier known to those skilled in the art may be used in the pharmaceutical composition. The choice of carrier may be appropriately determined, in part, based on the properties of the substance (i.e., cell or compound) to be administered.

Suitable carrier includes physiological saline, gelatin, water, alcohol, natural or synthetic oil, carbohydrate solution, glycol, injectable organic ester such as ethyl oleate, or combinations of these materials. The pharmaceutical composition may further contain preservative and/or other additives such as antimicrobial agent, antioxidant, chelating agent, and/or inert gas, and/or other active ingredients. In one embodiment of the present invention, a population of cardiomyocytes or proliferative cardiomyocytes is encapsulated according to known encapsulation techniques.

In one embodiment of the present invention, the population of cardiomyocytes or proliferative cardiomyocytes is present in a matrix.

The unit dosage form of the population of cardiomyocytes or proliferative cardiomyocytes may contain about 10³ to about 10⁹ cells, for example, about 10³ to about 10⁴ cells, about 10⁴ to about 10⁵ cells, about 10⁵ to about 10⁶ cells, about 10⁶ to about 10⁷ cells, about 10⁷ to about 10⁸ cells, or about 10⁸ to about 10⁹ cells, or the like.

In one embodiment of the present invention, a method for inducing myocardial regeneration in a population of cardiomyocytes in vitro and transplanting the population of cardiomyocytes into a target heart is provided. The population of cardiomyocytes can be used for allogeneic or autologous transplantation to an individual in need thereof.

### (Combination Therapy)

The terms "combination therapy" or "adjunctive therapy" when defining the use of FOXO6 protein, FOXO6 nucleic acid, or a vector containing FOXO6 nucleic acid together with one or more other medicaments, encompass the sequential administration of each medicament in a regimen that produces a beneficial effect of the combination of medicaments. The terms "combination therapy" and "adjunctive therapy" are intended to encompass the co-administration of these medicaments substantially simultaneously, such as in a single formulation having the above medicaments in a fixed ratio, or in multiple separate formulations of each medicament.

According to various embodiments of the present invention, one or more of FOXO6 protein, FOXO6 nucleic acid, and a vector containing FOXO6 nucleic acid may be formulated or administered in combination with one or more additional therapeutic agents. Therefore, according to various embodiments of the present invention, at least one of the FOXO6 protein, FOXO6 nucleic acid, and vector containing FOXO6 nucleic acid may be included in a combination therapy plan with surgery and/or other known treatment agents or therapeutic agents, and/or adjuvants or prophylactic agents.

In one embodiment of the present invention, FOXO6 is preferably used in combination with a cell cycle activator.

In the present specification, "cell cycle activator" is not particularly limited as long as it is a factor that positively activates the cell cycle and promotes proliferation. Therefore, the "cell cycle activator" includes factors that suppress (negatively activate) the cell cycle, in addition to factors that directly promote cell proliferation.

In one embodiment of the present invention, the cell cycle activator may be at least one selected from the group consisting of growth factors, growth factor receptors, intracellular factors with growth-promoting effects, cell cycle factors, anti-proliferation inhibitors, and molecules that activate these molecular mechanisms.

The growth factor is not particularly limited, and examples include NRG1, G-CSF, retinoic acid, TGF-β, IGF, PDGF, SHH, RLN3, FGF1, Periostin, and the like.

The growth factor receptor is not particularly limited, and examples include IGFR, PDGFR, SMO, PTCH, RXFP, CFTR, GP130, IL11R, FGFR, BMPR, ACVR, TGFR, EGFR, ERBB2, ERBB3, ERBB4, and the like.

Intracellular factors with proliferation-promoting effects are not particularly limited, and examples include PI3K, AKT, PLC, SOS, RAS, RAF, MEK, ERK, PKC, NFKB, STAT, SMAD, FOS, JUN, MYC, BCR-ABL, FOXM1, and the like.

Cell cycle factors are not particularly limited, and examples include cyclin (e.g., cyclin D, E, A, B), CDK (e.g., CDK6, 4, 2, 1), E2F (e.g., E2F1, 2, 3), and the like.

Anti-proliferation inhibitors refer to factors that inhibit the function of factors to inhibit proliferation. In other words, "anti-proliferation inhibitor" can be rephrased as "inhibitors of proliferation inhibitory factors". Antiproliferative inhibitors are not particularly limited, and examples include GSK3-β inhibitors, Hippo pathway inhibitors, inhibitors of cell cycle inhibitors (e.g., p15INK4B, p16INK4A, p21CIP1, p27KIP1, RB), and the like.

Modules that activate these molecular mechanisms are not particularly limited, and examples include Agrin, small molecule compounds (e.g., BIO, SC79, TC11, LNFPI), and the like.

Many medicaments are commercially available, undergoing clinical evaluation and preclinical development, and may be selected as part of a combination therapy for the treatment of the diseases and conditions recited above. Suitable medicaments that can be used in combination therapy can be recognized by those skilled in the art. Appropriate medicaments are listed, for example, in the Merck Index, An Encyclopedia of Chemicals, Drugs and Biologicals, 12th Ed., 1996, and subsequent editions, the entire contents of which are incorporated herein by reference.

The combination plan may include administering an additional medicament together, sequentially, or at intervals, as appropriate according to each situation. Furthermore, the combination of at least one FOXO6 protein, FOXO6 nucleic acid, or vector containing FOXO6 nucleic acid with additional medicaments may be synergistic. Additional medicaments are not particularly limited, and include various forms of medicaments such as nucleic acids, proteins, compounds, and the like. The additional medicaments may preferably be the cell cycle activators described above.

The co-administration of at least one of FOXO6 protein, FOXO6 nucleic acid, and a vector containing FOXO6 nucleic acid with an additional medicament may exert effects by the medicament being at the same unit dose as another active medicament, or one or more other medicaments may be present in individual unit doses to be administered at the same or similar time, or at different time, according to the administration plan or schedule. Sequential administration may be in any order as necessary. Especially when a cumulative or synergistic effect is desired, it may be required that the physiological effect of the first or initial compound is currently ongoing when the second or subsequent compounds are administered.

### (Others)

In addition to those mentioned above, another embodiment of the present invention also relates to the following.
[36] A method for treating heart disease comprising
   administering a therapeutically effective amount of FOXO6 to a subject.
[36a] The method of [36], wherein the aforementioned FOXO6 is administered to the aforementioned subject.
[36b] The method of [36] or [36a], wherein the aforementioned FOXO6 is administered to the heart of the aforementioned subject.
[36c] The method of any of [36] to [36b], which promotes heart regeneration of the aforementioned subject.
[36d] The method of [36c], wherein the aforementioned heart regeneration is characterized by an increase in ejection fraction, fractional shortening, or both.
[36e] The method of [36c] or [36d], wherein the aforementioned heart regeneration is characterized by an increase in vascular endothelial cells, epicardial cells, or both.
[36f] The method of any of [36] to [36e], wherein the aforementioned FOXO6 induces dedifferentiation of the aforementioned cardiomyocytes to produce proliferative cardiomyocytes.
[36g] The method of [36f], further comprising differentiating a population of the aforementioned proliferative cardiomyocytes to produce a population of cardiomyocytes.
[36h] The method of [36g], wherein the aforementioned differentiation occurs in the substantial absence of the aforementioned FOXO6 or after the induction of the aforementioned FOXO6 has ceased.
[36i] The method of any of [36] to [36h], wherein the aforementioned FOXO6 is FOXO6 protein, FOXO6 nucleic acid, or a vector containing FOXO6 nucleic acid.
[36j] The method of [36i], wherein the aforementioned FOXO6 protein is a protein comprising an amino acid sequence that is at least 80% identical to the amino acid sequence shown in SEQ ID NO: 1, SEQ ID NO: 3, SEQ ID NO: 5, or SEQ ID NO: 7.
[36k] The method of [36i], wherein the aforementioned FOXO6 nucleic acid is a protein that comprises or consists of a base sequence that is at least 80% identical to the base sequence shown in SEQ ID NO: 2, SEQ ID NO: 4, SEQ ID NO: 6, or SEQ ID NO: 8.
[36l] The method of [36i], wherein the aforementioned vector is a FOXO6 nucleic acid operatively bound to a promoter.
[36m] The method of [36l], wherein the aforementioned promoter is a cardiac-specific promoter.
[36n] The method of [36m], wherein the aforementioned cardiac-specific promoter is selected from the group consisting of an α-myosin heavy chain (α-MHC) promoter, a myosin light chain 2 (MLC-2) promoter, a cardiac troponin C (cTnC) promoter, an NCX1 promoter, and a TNNT2 promoter.
[36o] The method of [36l] or [36m], wherein the aforementioned promoter is an inducible promoter.
[36p] The method of [36o], wherein the aforementioned inducible promoter is selected from the group consisting of tetracycline inducible promoter, steroid hormone inducible promoter, or hypoxia-inducible promoter, a promoter specific to the region near the site of heart injury, or a promoter that responds to stress resulting from ischemia and reperfusion.
[36q] The method of any of [36] to [36p], further comprising administering a cell cycle activator.
[36r] The method of [36] or [36p], which is used in combination with a cell cycle activator.
[36s] The method of [36q] or [36r], wherein the aforementioned cell cycle activator is at least one selected from the group consisting of a growth factor, a growth factor receptor, an intracellular factor with a growth-promoting effect, a cell cycle factor, an anti-proliferation inhibitor, and a molecule that activates molecular mechanisms of these.
[36t] The method of [36q] or [36r], wherein the aforementioned cell cycle activator is at least one selected from the group consisting of NRG1, G-CSF, retinoic acid, TGF-β, IGF, PDGF, SHH, RLN3, FGF1, Periostin, Agrin, a GSK3-β inhibitor, and a Hippo pathway inhibitor.
[36u] The method of any of [36f] to [36t], wherein the aforementioned proliferative cardiomyocyte is a cardiomyocyte progenitor cell, an immature cardiomyocyte, or a cardiomyocyte having an embryonic phenotype.
[36v] The method of any of [36] to [36u], wherein the aforementioned treatment of heart disease involves myocardial regeneration, and the myocardial regeneration does not involve reprogramming of the cardiomyocyte cell lineage.
[36w] The method of any of [36f] to [36t], wherein the aforementioned proliferative cardiomyocyte re-enters the cell cycle.
[36x] The method of any of [36] to [36w], wherein the aforementioned treatment of heart disease involves myocardial regeneration, and the myocardial regeneration is characterized by an increase in the number of epicardial cells, vascular endothelial cells, or both.
[36y] The method of any of [36] to [36x], wherein the aforementioned subject has heart disease associated with the loss of cardiomyocytes.
[36z] The method of [36y], wherein the aforementioned heart disease is myocardial infarction, ischemic cardiomyopathy, dilated cardiomyopathy, or heart failure.

In another embodiment, the present invention also relates to the following.
[37] Use of FOXO6 in the manufacture of a medicament for the treatment of heart disease.
[37a] The use of [37], wherein the aforementioned FOXO6 is administered to the subject.
[37b] The use of [37] or [37a], wherein the aforementioned FOXO6 is administered to the heart of the subject.
[37c] The use of any of [37] to [37b], which promotes heart regeneration of the subject.
[37d] The use of [37c], wherein the aforementioned heart regeneration is characterized by an increase in ejection fraction, fractional shortening, or both.
[37e] The use of [37c] or [37d], wherein the aforementioned heart regeneration is characterized by an increase in vascular endothelial cells, epicardial cells, or both.
[37f] The use of any of [37] to [37e], wherein the aforementioned FOXO6 induces dedifferentiation of the aforementioned cardiomyocytes to produce proliferative cardiomyocytes.
[37g] The use of [37f], further comprising differentiating a population of the aforementioned proliferative cardiomyocytes to produce a population of cardiomyocytes.
[37h] The use of [37g], wherein the aforementioned differentiation occurs in the substantial absence of the aforementioned FOXO6 or after the induction of the aforementioned FOXO6 has ceased.
[37i] The use of any of [37] to [37h], wherein the aforementioned FOXO6 is FOXO6 protein, FOXO6 nucleic acid, or a vector containing FOXO6 nucleic acid.
[37j] The use of [37i], wherein the aforementioned FOXO6 protein is a protein comprising an amino acid sequence that is at least 80% identical to the amino acid sequence shown in SEQ ID NO: 1, SEQ ID NO: 3, SEQ ID NO: 5, or SEQ ID NO: 7.
[37k] The use of [37i], wherein the aforementioned FOXO6 nucleic acid is a protein that comprises or consists of a base sequence that is at least 80% identical to the base sequence shown in SEQ ID NO: 2, SEQ ID NO: 4, SEQ ID NO: 6, or SEQ ID NO: 8.
[37l] The use of [37i], wherein the aforementioned vector is a FOXO6 nucleic acid operatively bound to a promoter.
[37m] The use of [37l], wherein the aforementioned promoter is a cardiac-specific promoter.
[37n] The use of [37m], wherein the aforementioned cardiac-specific promoter is selected from the group consisting of an α-myosin heavy chain (α-MHC) promoter, a myosin light chain 2 (MLC-2) promoter, a cardiac troponin C (cTnC) promoter, an NCX1 promoter, and a TNNT2 promoter.
[37o] The use of [37l] or [37m], wherein the aforementioned promoter is an inducible promoter.
[37p] The use of [37o], wherein the aforementioned inducible promoter is selected from the group consisting of tetracycline inducible promoter, steroid hormone inducible promoter, or hypoxia-inducible promoter, a promoter specific to the region near the site of heart injury, or a promoter that responds to stress resulting from ischemia and reperfusion.
[37q] The use of any of [37] to [37p], further comprising a cell cycle activator.
[37r] The use of [37] or [37p], which is used in combination with a cell cycle activator.
[37s] The use of [37q] or [37r], wherein the aforementioned cell cycle activator is at least one selected from the group consisting of a growth factor, a growth factor receptor, an intracellular factor with a growth-promoting effect, a cell cycle factor, an anti-proliferation inhibitor, and a molecule that activates molecular mechanisms of these.
[37t] The use of [37q] or [37r], wherein the aforementioned cell cycle activator is at least one selected from the group consisting of NRG1, G-CSF, retinoic acid, TGF-β, IGF, PDGF, SHH, RLN3, FGF1, Periostin, Agrin, a GSK3-β inhibitor, and a Hippo pathway inhibitor.
[37u] The use of any of [37f] to [37t], wherein the aforementioned proliferative cardiomyocyte is a cardiomyocyte progenitor cell, an immature cardiomyocyte, or a cardiomyocyte having an embryonic phenotype.
[37v] The use of any of [37] to [37u], wherein the aforementioned treatment of heart disease involves myocardial regeneration, and the myocardial regeneration does not involve reprogramming of the cardiomyocyte cell lineage.
[37w] The use of any of [37f] to [37t], wherein the aforementioned proliferative cardiomyocyte re-enters the cell cycle.
[37x] The use of any of [37] to [37w], wherein the aforementioned treatment of heart disease involves myocardial regeneration, and the myocardial regeneration is characterized by an increase in the number of epicardial cells, vascular endothelial cells, or both.
[37y] The use of any of [37] to [37x], wherein the aforementioned subject has heart disease associated with the loss of cardiomyocytes.
[37z] The use of [37y], wherein the aforementioned heart disease is myocardial infarction, ischemic cardiomyopathy, dilated cardiomyopathy, or heart failure.

In another embodiment, the present invention also relates to the following.
[38] FOXO6 for use in the treatment of heart disease.
[38a] The FOXO6 of [38], wherein the aforementioned FOXO6 is administered to the subject.
[38b] The FOXO6 of [38] or [38a], wherein the aforementioned FOXO6 is administered to the heart of the subject.
[38c] The FOXO6 of any of [38] to [38b], which promotes heart regeneration of the subject.
[38d] The FOXO6 of [38c], wherein the aforementioned heart regeneration is characterized by an increase in ejection fraction, fractional shortening, or both.
[38e] The FOXO6 of [38c] or [38d], wherein the aforementioned heart regeneration is characterized by an increase in vascular endothelial cells, epicardial cells, or both.
[38f] The FOXO6 of any of [38] to [38e], wherein the aforementioned FOXO6 induces dedifferentiation of the aforementioned cardiomyocytes to produce proliferative cardiomyocytes.
[38g] The FOXO6 of [38f], further comprising differentiating a population of the aforementioned proliferative cardiomyocytes to produce a population of cardiomyocytes.
[38h] The FOXO6 of [38g], wherein the aforementioned differentiation occurs in the substantial absence of the aforementioned FOXO6 or after the induction of the aforementioned FOXO6 has ceased.
[38i] The FOXO6 of any of [38] to [38h], wherein the aforementioned FOXO6 is FOXO6 protein, FOXO6 nucleic acid, or a vector containing FOXO6 nucleic acid.
[38j] The FOXO6 of [38i], wherein the aforementioned FOXO6 protein is a protein comprising an amino acid sequence that is at least 80% identical to the amino acid sequence shown in SEQ ID NO: 1, SEQ ID NO: 3, SEQ ID NO: 5, or SEQ ID NO: 7.
[38k] The FOXO6 of [38i], wherein the aforementioned FOXO6 nucleic acid is a protein that comprises or consists of a base sequence that is at least 80% identical to the base sequence shown in SEQ ID NO: 2, SEQ ID NO: 4, SEQ ID NO: 6, or SEQ ID NO: 8.
[38l] The FOXO6 of [38i], wherein the aforementioned vector is a FOXO6 nucleic acid operatively bound to a promoter.
[38m] The FOXO6 of [38l], wherein the aforementioned promoter is a cardiac-specific promoter.
[38n] The FOXO6 of [38m], wherein the aforementioned cardiac-specific promoter is selected from the group consisting of an α-myosin heavy chain (α-MHC) promoter, a myosin light chain 2 (MLC-2) promoter, a cardiac troponin C (cTnC) promoter, an NCX1 promoter, and a TNNT2 promoter.
[38o] The FOXO6 of [38l] or [38m], wherein the aforementioned promoter is an inducible promoter.
[38p] The FOXO6 of [38o], wherein the aforementioned inducible promoter is selected from the group consisting of tetracycline inducible promoter, steroid hormone inducible promoter, or hypoxia-inducible promoter, a promoter specific to the region near the site of heart injury, or a promoter that responds to stress resulting from ischemia and reperfusion.
[38q] The FOXO6 of [38] or [38p], which is used in combination with a cell cycle activator.
[38r] The FOXO6 of [38q], wherein the aforementioned cell cycle activator is at least one selected from the group consisting of a growth factor, a growth factor receptor, an intracellular factor with a growth-promoting effect, a cell cycle factor, an anti-proliferation inhibitor, and a molecule that activates molecular mechanisms of these.
[38s] The FOXO6 of [38q], wherein the aforementioned cell cycle activator is at least one selected from the group consisting of NRG1, G-CSF, retinoic acid, TGF-β, IGF, PDGF, SHH, RLN3, FGF1, Periostin, Agrin, a GSK3-β inhibitor, and a Hippo pathway inhibitor.
[38t] The FOXO6 of any of [38f] to [38s], wherein the aforementioned proliferative cardiomyocyte is a cardiomyocyte progenitor cell, an immature cardiomyocyte, or a cardiomyocyte having an embryonic phenotype.
[38u] The FOXO6 of any of [38] to [38t], wherein the aforementioned treatment of heart disease involves myocardial regeneration, and the myocardial regeneration does not involve reprogramming of the cardiomyocyte cell lineage.
[38v] The FOXO6 of any of [38f] to [38s], wherein the aforementioned proliferative cardiomyocyte re-enters the cell cycle.
[38w] The FOXO6 of any of [38] to [38v], wherein the aforementioned treatment of heart disease involves myocardial regeneration, and the myocardial regeneration is characterized by an increase in the number of epicardial cells, vascular endothelial cells, or both.
[38x] The FOXO6 of any of [38] to [38w], wherein the aforementioned subject has heart disease associated with the loss of cardiomyocytes.
[38y] The FOXO6 of [38x], wherein the aforementioned heart disease is myocardial infarction, ischemic cardiomyopathy, dilated cardiomyopathy, or heart failure.

In another embodiment, the present invention relates to the following.
A method for treating heart disease in a subject, comprising administering a therapeutically effective amount of the population of cardiomyocytes or proliferative cardiomyocytes of [34], or the composition of [35] to the subject.

In another embodiment, the present invention relates to the following.
Use of the population of cardiomyocytes or proliferative cardiomyocytes of [34], or the composition of [35], in the manufacture of a medicament for the treatment of heart disease.

In another embodiment, the present invention also relates to the following.

The population of cardiomyocytes or proliferative cardiomyocytes of [34], or the composition of [35], for use in the treatment of heart disease.

### [Example]

The present invention is described in more detail below using examples, by which the scope of the present invention is not limited. References cited throughout the present specification are incorporated entirely by reference.

### (1. Expression of foxo6b gene in Klf1-expressing heart)

To investigate the genes expressed in hearts overexpressing Klf1, according to the method described in Ogawa et al. Science 2021, total RNA was extracted and a library was prepared, and then the sequence of expressed genes was analyzed using a sequencer.

The results are shown in Fig. 1. As shown in Fig. 1, it was found that in hearts where myocardial regeneration was strongly induced by overexpression of Klf1, the expression of foxo6b was remarkably increased.

### (2. Regulation of foxo6b gene expression by Klf1)

To investigate whether Klf1 is involved in the expression of the foxo6b gene, the following experiment was performed.

### (ChIP-seq)

Analysis was performed using gene-transformed zebrafish capable of inducing the expression of Klf1 (HA-Klf1) with a 3×HA-tag, specifically in cardiac muscle.

In adult zebrafish, analysis was performed using the heart 7 days after induction of HA-Klf1 expression (with Klf1), combining chromatin immunoprecipitation (ChIP) using anti-HA antibodies (ab9110, Abcam) and next-generation sequencing (ChIP-seq). ChIP chromatin was fragmented using an ultrasound generator (Covaris M220) and recovered using the SimpleChIP Plus Sonication Chromatin IP kit (Cell Signaling Technology). Hearts without Klf1 expression induction were used as a control (without Klf1).

The results are shown in Fig. 2A. This analysis allows for the identification of genomic regions to which Klf1 directly binds in cardiomyocytes. The analysis revealed binding to the expression regulatory region upstream of the foxo6b gene (black boxes 1 and 2) and to the intron region (black box 3).

### (Quantitative PCR analysis)

Quantitative PCR analysis was performed using chromatin immunoprecipitation samples (Klf1-ChIP) using anti-HA antibody (Fig. 2B). Power SYBR PCR Master Mix (Thermo Fisher) was used. PCR primers were designed to sandwich each Klf1 binding region (black box 1: SEQ ID NOs. 9 and 10, black box 2: SEQ ID NOs. 11 and 12, black box 3: SEQ ID NOs. 13 and 14). ChIP samples prepared using isotype control antibody (2729, Cell Signaling Technology) were used as negative control (control). Statistical analysis was performed using unpaired t-test.

The results are shown in Fig. 2B. All detected Klf1 binding regions (black boxes 1-3 in Fig. 2A) were amplified, confirming that Klf1 binds to the binding regions identified by ChIP-seq analysis.

The sequences of the primers used in this Example are shown in Table 1 below.

**[Table 1]**

| ID | **sequence** | **SEQ ID NO** |
|---|---|---|
| ChIP-PCR-F1 | agtcaaagcatagtatgttgccaaccca | 9 |
| ChlP-PCR-R1 | atgtggcctcccaatgtcaccgcatgtga | 10 |
| ChIP-PCR-F2 | tcaataggatctgatacgcgcgttctggct | 11 |
| ChIP-PCR-R2 | tggcaagcgtttctcttgtctatgaacaagt | 12 |
| ChIP-PCR-F3 | tttgctctatccgtccatccttcact | 13 |
| ChIP-PCR-R3 | tgctgggcgagccgggccaagttccct | 14 |
| foxo6b-F | gatcacgcgtgccattgaga | 15 |
| foxo6b-R | gtgtctaatggagttcttccagc | 16 |
| foxo6b-tagman | atgactggatggtgcgatttgtgc | 17 |
| rpl13a-F | tctggaggactgtaagaggtatgc | 18 |
| rpl13a-R | agacgcacaatcttgagagcag | 19 |
| F1 | gagtctgtgcgatctcccacaggtgtctgtactct | 20 |
| R1 | tgctgggcgagccgggccaagttccct | 21 |
| F2 | tttgctctatccgtccatccttcact | 22 |
| R2 | tcgatggtgatgcttggcaattcgggt | 23 |
| F3 | agagcattattgtaggaaatgtattgtatatgtacattctgtgca | 24 |
| R3 | attgtcagtggacgctgtctttcggatgagacgtt | 25 |
| vmhcl-F | tgcgaagtctgaggcacgta | 26 |
| vmhcl-R | ggttgtcttgctccgcttgc | 27 |
| actc1a-F | ttgcgctacaactttgaaccatc | 28 |
| actcla-R | gacagcacgtggagcatcat | 29 |
| myom1b-F | gaccacaggcgagagctatgt | 30 |
| myom1b-R | gcaggtgaagcgatagcagc | 31 |
| tbx5b-F | accagaaccacaccatcactca | 32 |
| tbx5b-R | ttggtgtacgggacatgcga | 33 |
| mef2ca-F | attttggggctgattcgccatc | 34 |
| mef2ca-R | ttcgtttggcaccagtccg | 35 |
| nkx2-5-F | aaagacatcgggttttgtcaggaa | 36 |
| nkx2-5-R | acacctgcgcctgagagaag | 37 |
| foxo6bSA-F1 | aaaaccggtgccaccatgtacccatacgatgttcctgac | 38 |
| foxo6bSA-R1 | cttcaggtacttagtgccattgtccatagctacagctcgtctgcggggtccttt | 39 |
| foxo6bSA-F2 | aaaggaccccgcagacgagctgtagctatggacaatggcactaagtacctgaag | 40 |
| foxo6bSA-R2 | aaaaaagcggccgcttacccaggcacccaactctggctatt | 41 |

### (3. Expression of foxo6b gene in damaged zebrafish heart)

The following experiment was performed to investigate the expression of the foxo6b gene in damaged zebrafish hearts.

### (Quantitative PCR Analysis)

The expression of the foxo6b gene in adult zebrafish hearts 7 days after injury was measured by quantitative PCR. Heart injury was induced using a myocardial-specific removal model based on the method described in Wang et al. Development 2011. Uninjured hearts were used as a control. TaqMan Universal PCR Master Mix (Thermo Fisher) and Power SYBR PCR Master Mix (Thermo Fisher) were used for PCR. As primers and TaqMan probes, those shown in SEQ ID NOs. 15-19 were used.

The results are shown in Fig. 3A. Compared to uninjured hearts (control), a significant increase in foxo6b mRNA expression was observed in hearts 7 days after injury.

### (In situ hybridization method)

Foxo6b mRNA expression was analyzed using a highly sensitive in situ hybridization method (RNAscope) on heart sections 7 days after injury. Cardiomyocytes were detected by immunofluorescence staining using an anti-troponin antibody (GTX127379, GeneTex). RNAscope chromogenic RED (Advanced Cell Diagnostics) was used for color development of RNAscope signal.

The results are shown in Fig. 3B. Expression of Foxo6b mRNA was not observed in the uninjured heart (control), but foxo6b mRNA expression was observed in cardiomyocytes (troponin) in the heart 7 days after injury (indicated by the arrow in Fig. 3B). This result was consistent with the results of quantitative PCR analysis.

### (4. Production of time- and tissue-specific foxo6b gene expression suppression model)

A model (zebrafish) for suppressing the expression of the foxo6b gene in a time- and tissue-specific manner was produced.

Specifically, a line was established in which a gene trap cassette (Zwitch2) that causes Cre-dependent inversion was inserted into the first intron of the zebrafish foxo6b gene. Homologous recombination by genome editing, as described in Ogawa et al. Science 2021, was used for the insertion of Zwitch2. The Zwitch2 cassette consists of the loxP/lox5171 sequence, a splice acceptor site, a transcription stop sequence (Stop), and a lens-specific GFP expression cassette (lensGFP) for strain screening (Fig. 4A).

In this model, normal splicing occurs between exon 1 and exon 2 in the absence of Cre activity. However, Cre activity causes recombination between loxP and lox5171, leading to forward inversion of the splice acceptor site and trapping of the upstream exon, thereby interrupting foxo6b mRNA transcription (Fig. 4B). In Fig. 4B, the lens GFP cassette was omitted to simplify the illustration of the gene expression suppression mechanism.

In zebrafish lines with the Zwitch2 cassette inserted, lens-specific GFP expression was observed (Fig. 4C). Furthermore, in this model, insertion of Zwitch2 was confirmed by genomic PCR (Fig. 4D). As the primers, those shown by SEQ ID NOs. 20-25 were used.

### (5. Decrease in cardiomyocyte dedifferentiation and proliferation by suppression of cardiomyocyte-specific foxo6b gene expression)

To investigate the influence of suppressing cardiomyocyte-specific foxo6b gene expression on cardiomyocytes, the following experiment was conducted.

### (Immunofluorescence staining: dedifferentiation marker)

The zebrafish line Tg (cmlc2: CreER), which expresses tamoxifen-dependent Cre (CreER) specifically in cardiomyocytes, was crossed with the foxo6bZwitch2/+ line to obtain Tg(cmlc2: CreER); foxo6b^{Zwitch2/Zwitch2} line and the foxo6b^{Zwitch2/Zwitch2} line (control). After growth of each line, tamoxifen was administered, and after lapse of sufficient time, cryo-injury was induced in the cardiac apex. The level of dedifferentiation was analyzed using ventricular sections 7 days after injury. Sm22 (Smooth muscle actin 22 alpha) was used as a dedifferentiation marker, and immunofluorescence staining was performed using an anti-Sm22 antibody (GTX125994, GeneTex). For fluorescence staining of cardiomyocytes, an anti-myosin heavy chain antibody (sc-32732, Santa Cruz) was used.

The results are shown in Fig. 5A. In the control group, Sm22 expression was observed in the cardiomyocytes at the injury periphery where regeneration occurred (dotted line) (inset, arrowhead), but in hearts where foxo6b expression was suppressed, Sm22 expression was reduced (inset, arrowhead). The graph on the right shows the quantitative results thereof (Mann-Whitney U test).

### (Immunofluorescence staining: cell proliferation marker)

Analysis of cardiomyocyte proliferation using ventricular sections 7 days after injury. As a proliferation marker, PCNA (proliferation cell nuclear antibody) was used, and Mef2 was used as a cardiomyocyte nuclear marker. Immunofluorescence staining was performed using anti-PCNA antibody (P8825, Sigma Aldrich) and anti-Mef2 antibody (sc-313, SantaCruz).

The results are shown in Fig. 5B. In the control group, co-stained image of Mef2 and PCNA, indicating proliferating cardiomyocytes, was observed in the cardiomyocytes at the injury periphery where regeneration occurs (dotted line) (inset, arrow). However, it was found that the co-stained image decreased in hearts with suppressed foxo6b expression (inset, arrow). As the nuclear marker for all cells, DAPI (4',6-diamidino-2-phenyllindole) was used. The graph on the right shows the quantitative results thereof (Mann-Whitney U test).

### (6. Myocardial regeneration failure due to myocardial-specific foxo6b gene expression suppression)

The influence of myocardial-specific foxo6b gene expression suppression on myocardial regeneration was investigated. Specifically, myocardial injury was induced using the same method as in 5. above. Myocardial regeneration was analyzed using frozen ventricular sections 45 days after injury by Picro-Mallory staining.

The results are shown in Fig. 6. Remarkable myocardial regeneration was observed in the control ventricle 45 days after injury (left Figure). On the other hand, in hearts where myocardial-specific foxo6b gene expression was suppressed, similar myocardial regeneration was not observed, and scarring accompanied by collagen deposition was observed (right Figure). Therefore, it was shown that suppression of myocardial-specific foxo6b gene expression leads to myocardial regeneration failure.

### (7. Production of constitutively activated foxo6b-expressing zebrafish line)

A constitutively activated foxo6b (foxo6bSA) was produced by replacing serine 199, which is phosphorylated by the PI3K-Akt pathway, with alanine (Fig. 7A). Specifically, a sequence in which the base sequence of serine 199 was replaced with alanine was first amplified using overlap extension PCR, and then fragmented using restriction enzymes AgeI and NotI. Then, this obtained partial sequence was inserted into the AgeI and NotI cleavage sites of the wild-type foxo6b sequence to produce a foxo6bSA mutant. As the primers, those shown in SEQ ID NOs. 38-41 were used.

Furthermore, a line Tg (actb-LSL-foxo6bSA) expressing foxo6bSA only when Cre is activated downstream of the beta-actin promoter (β-actin2) was established and crossed with Tg (cmlc2:CreER) (Fig. 7B). Tg (actb-LSL-foxo6bSA) was produced by inserting foxo6bSA cDNA into a vector containing the β-actin2 promoter and LSL cassette, and injecting the obtained construct into one-cell stage zebrafish embryos. As the injection method into zebrafish embryos, strain establishment method, and vector, those described in Ogawa et al. Science 2021 were used.

The Tg (cmlc2:CreER) used was the line described in Kikuchi et al. Nature 2010. In the dual line obtained by the above-mentioned crossing, foxo6bSA expression can be induced specifically in the cardiomyocyte only upon administration of tamoxifen.

### (8. Induction of cardiomyocyte dedifferentiation by foxo6bSA expression)

To investigate the influence of foxo6bSA expression on cardiomyocyte dedifferentiation, the following experiment was performed.

### (Immunofluorescence staining)

Tg (actb-LSL-foxo6bSA) (control, foxo6bSA-OFF) and Tg (cmlc2:CreER; actb-LSL-foxo6bSA) (foxo6bSA-ON) were treated with tamoxifen, and the hearts were analyzed after 7 days. As a dedifferentiation marker, Sm22 was used, and immunofluorescence staining was performed using an anti-Sm22 antibody (GTX125994, GeneTex). Cardiomyocyte sarcomere was stained using an anti-actinin antibody (A7811, Sigma Aldrich). For fluorescent staining of cardiomyocytes, an anti-myosin heavy chain antibody (sc-32732, SantaCruz) was used. DAPI was used as a nuclear staining marker for all cells. Magnified, single-stained images of the areas within the white-lined squares are shown to the right of each panel.

The results are shown in Fig. 8A. Extremely pronounced Sm22 expression and sarcomere pattern disruption were observed in the foxo6bSA-ON heart, and it was found that cardiomyocyte dedifferentiation was strongly induced in this heart.

### (Quantitative PCR analysis)

Quantitative PCR analysis (unpaired t-test) of sarcomere-related genes and cardiomyocyte differentiation-related transcription factors was performed using the same heart samples as above. As the primers, those shown in SEQ ID NOs. 18, 19, and 26-37 were used.

The results are shown in Fig. 8B. The left graph shows the results of sarcomere-related genes, and the right graph shows the results of cardiomyocyte differentiation-related transcription factors. Remarkably reduced expression of sarcomere-related genes and cardiomyocyte differentiation-related transcription factors was observed in foxo6bSA-ON hearts, and it was found that cardiomyocyte dedifferentiation was strongly induced in these hearts.

### (9. Influence of foxo6bSA expression on cardiomyocyte proliferation)

To investigate the influence of foxo6bSA expression on cardiomyocyte proliferation, the following experiment was performed.

### (Immunofluorescence staining: EdU analysis)

Tg (actb-LSL-foxo6bSA; cmlc2:GFP) (control, foxo6bSA-OFF) and Tg (cmlc2:CreER; actb-LSL-foxo6bSA; cmlc2:GFP) (foxo6bSA-ON) were treated with tamoxifen, and the hearts were analyzed 7 days later. EdU (ethynyl deoxyuridine) was used as the S-phase marker. For EdU analysis, 5 µL of 8 mM EdU was administered intraperitoneally 5, 6, and 7 days after tamoxifen administration. EdU staining was performed using the Click-iT EdU imaging kit (Thermo Fisher). Cardiomyocyte-specific GFP expression was detected using an anti-GFP antibody (ab13970, Abcam). DAPI was used as a nuclear staining marker for all cells.

The results are shown in Fig. 9A. Analysis of images by confocal microscope revealed that EdU-positive cells were stromal cells, and no uptake by cardiomyocytes was observed.

### (Immunofluorescence staining: phosphorylated histone H3)

Using the same heart samples as above, immunofluorescence staining for an M-phase marker, phosphorylated histone H3, was performed. Anti-phospho-Histone H3 (pHH3) antibody (06-570, Merck Millipore) and anti-GFP antibody (ab13970, Abcam) were used.

The results are shown in Fig. 9B. Phospho-Histone H3 staining was not observed in cardiomyocytes. The graph on the right shows the quantitative results thereof (unpaired t-test).

### (10. Influence of co-expression of foxo6bSA and ccnd1 on cardiomyocyte proliferation)

To investigate the influence of co-expression of foxo6bSA and ccnd1 on cardiomyocyte proliferation, the following experiment was performed.

### (Immunofluorescence staining: phosphorylated histone H3)

Tg (actb-LSL-foxo6bSA) (foxo6bSA-OFF), Tg (cmlc2:CreER; actb-LSL-foxo6bSA) (foxo6bSA-ON), Tg (actb-LSL-ccnd1) (ccnd1-OFF), and Tg (cmlc2:CreER; actb-LSL-ccnd1) (ccnd1-ON) were treated with tamoxifen, and the hearts were analyzed 5 days later. Cardiomyocytes in the division phase were identified by immunofluorescence staining using anti-phospho-Histone H3 (pHH3) antibody (06-570, Merck Millipore) and anti-Actinin antibody (A7811, Sigma Aldrich).

The results are shown in Fig. 10. In Fig. 10, arrows indicate mitotic cardiomyocytes. Co-expression of foxo6bSA and ccnd1 showed higher cardiomyocyte proliferation compared to the control, foxo6bSA alone, and ccnd1 alone. The graph on the right shows the quantitative results thereof (unpaired t-test).

### (11. Influence of co-expression of foxo6SA and ccnd1 on cardiomyocyte proliferation)

To investigate the influence of co-expression of foxo6SA and ccnd1 on cardiomyocyte proliferation, the following experiment was performed.

### (Immunofluorescence staining: Aurora kinase B analysis)

FOXO6 and CCND1, which are human homologs of zebrafish Foxo6b and Ccnd1, were cloned. Constitutively activated FOXO6SA, similar to that in zebrafish, was produced, and mRNAs of FOXO6SA and CCND1 were synthesized. For the purpose of labeling expressing cells, fluorescent reporter genes (TagRFP, EGFP) were attached to these mRNAs via a 2A sequence. These mRNAs were introduced into human iPS cell-derived cardiomyocytes (hiPSC-CMs) and analyzed 4 days later. EGFP mRNA and TagRFP mRNA were introduced into the control group.

Immunofluorescence staining for a cell division marker Aurora kinase B was performed. Cardiomyocytes in the cell division phase were identified by immunofluorescence staining using anti-Aurora kinase B antibody (A5102, Sigma-Aldrich) and anti-Actinin antibody (A7811, Sigma-Aldrich). Nuclear staining was performed using DRAQ5 (ab108410, Abcam).

The results are shown in Fig. 11. In Fig. 11, arrows indicate cardiomyocytes in the division phase. Co-expression of FOXO6SA and CCND1 significantly increased cardiomyocyte division by about 2.5-fold compared to the control group. Fig. 11C shows the quantification results thereof (unpaired t-test).

### [Industrial Applicability]

The present invention is useful in the fields where dedifferentiation of cardiomyocytes is desired, particularly in the field of myocardial regeneration therapy. This application is based on a patent application No. 2023-179125 filed in Japan (filing date: October 17, 2023), the contents of which are incorporated in full herein.

## Claims

1. A composition for inducing myocardial regeneration comprising FOXO6 as an active ingredient.

2. The composition according to claim 1, wherein the FOXO6 is administered in a therapeutically effective amount to cardiomyocytes.

3. The composition according to claim 2, wherein the cardiomyocytes are those of an infant, child, or adult.

4. The composition according to claim 2, wherein the administration is performed in vitro.

5. The composition according to claim 2, wherein the cardiomyocytes are present in a subject.

6. The composition according to claim 5, wherein the FOXO6 is administered to the subject.

7. The composition according to claim 5, wherein the FOXO6 is administered to the heart of the subject.

8. The composition according to claim 5, wherein the myocardial regeneration promotes cardiac regeneration in the subject.

9. The composition according to claim 8, wherein the heart regeneration is **characterized by** an increase in ejection fraction, fractional shortening, or both.

10. The composition according to claim 8, wherein the heart regeneration is **characterized by** an increase in vascular endothelial cells, epicardial cells, or both.

11. The composition according to claim 2, wherein the FOXO6 induces dedifferentiation of the cardiomyocytes to produce proliferative cardiomyocytes.

12. The composition according to claim 11, further comprising differentiating a population of the proliferative cardiomyocytes to produce a population of cardiomyocytes.

13. The composition according to claim 12, wherein the differentiation occurs in the substantial absence of the FOXO6 or after the induction of the FOXO6 has ceased.

14. The composition according to claim 1, wherein the FOXO6 is FOXO6 protein, FOXO6 nucleic acid, or a vector containing FOXO6 nucleic acid.

15. The composition according to claim 14, wherein the FOXO6 protein is a protein comprising an amino acid sequence that is at least 80% identical to the amino acid sequence shown in SEQ ID NO: 1, SEQ ID NO: 3, SEQ ID NO: 5, or SEQ ID NO: 7.

16. The composition according to claim 14, wherein the FOXO6 nucleic acid is a nucleic acid that comprises or consists of a base sequence that is at least 80% identical to the base sequence shown in SEQ ID NO: 2, SEQ ID NO: 4, SEQ ID NO: 6, or SEQ ID NO: 8.

17. The composition according to claim 14, wherein the vector comprises FOXO6 nucleic acid operatively bound to a promoter.

18. The composition according to claim 17, wherein the promoter is a cardiac-specific promoter.

19. The composition according to claim 18, wherein the cardiac-specific promoter is selected from the group consisting of an α-myosin heavy chain (α-MHC) promoter, a myosin light chain 2 (MLC-2) promoter, a cardiac troponin C (cTnC) promoter, an NCX1 promoter, and a TNNT2 promoter.

20. The composition according to claim 17, wherein the promoter is an inducible promoter.

21. The composition according to claim 20, wherein the inducible promoter is selected from the group consisting of a tetracycline-inducible promoter, a steroid hormone-inducible promoter, a hypoxia-inducible promoter, a promoter specific to a region near a site of heart injury, or a promoter that responds to stress resulting from ischemia and reperfusion.

22. The composition according to claim 1, further comprising a cell cycle activator.

23. The composition according to claim 1, which is used in combination with a cell cycle activator.

24. The composition according to claim 22 or 23, wherein the cell cycle activator is at least one selected from the group consisting of a growth factor, a growth factor receptor, an intracellular factor with a growth-promoting effect, a cell cycle factor, an anti-proliferation inhibitor, and a molecule that activates molecular mechanisms of these.

25. The composition according to claim 22 or 23, wherein the cell cycle activator is at least one selected from the group consisting of NRG1, G-CSF, retinoic acid, TGF-β, IGF, PDGF, SHH, RLN3, FGF1, Periostin, Agrin, a GSK3-β inhibitor, and a Hippo pathway inhibitor.

26. The composition according to claim 11, wherein the proliferative cardiomyocyte is a cardiomyocyte progenitor cell, an immature cardiomyocyte, or a cardiomyocyte having an embryonic phenotype.

27. The composition according to claim 1, wherein the myocardial regeneration does not involve reprogramming of a cardiomyocyte cell lineage.

28. The composition according to claim 11, wherein the proliferative cardiomyocyte re-enters the cell cycle.

29. The composition according to claim 5, wherein the myocardial regeneration is **characterized by** an increase in the number of epicardial cells, vascular endothelial cells, or both.

30. The composition according to claim 5, wherein the subject has heart disease associated with the loss of cardiomyocytes.

31. The composition according to claim 30, wherein the heart disease is myocardial infarction, ischemic cardiomyopathy, dilated cardiomyopathy, or heart failure.

32. A method for inducing myocardial regeneration, comprising administering a therapeutically effective amount of FOXO6 to cardiomyocytes or inducing the expression of FOXO6 in cardiomyocytes.

33. The method according to claim 32, further comprising administering a therapeutically effective amount of a cell cycle activator to cardiomyocytes.

34. A population of cardiomyocytes or proliferative cardiomyocytes produced by the method according to claim 32 or 33.

35. A composition comprising cardiomyocytes, proliferative cardiomyocytes, or both produced by the method according to claim 32 or 33.

36. A method for treating heart disease, comprising administering a therapeutically effective amount of FOXO6 to a subject.

37. Use of FOXO6 in the manufacture of a medicament for the treatment of heart disease.

38. FOXO6 for use in the treatment of heart disease.

39. A method for inducing dedifferentiation of cardiomyocytes, comprising administering FOXO6 to cardiomyocytes or inducing the expression of FOXO6 in cardiomyocytes.
